# FASCICULE DE BREVET EUROPEEN

(11) **EP 0 755 447 B1**
(45) Date de publication et mention de la délivrance du brevet: **02.07.2003**
(21) Numéro de dépôt: 95918030.8
(22) Date de dépôt: 19.04.1995
(51) Int. Cl.: C12N 15/31, C12N 15/62, C07K 14/235, C12N 1/21, A61K 39/10

(54) **PROTEINES RECOMBINANTES DE L'HEMAGGLUTININE FILAMENTEUSE DE BORDETELLA, NOTAMMENT BORDETELLA PERTUSSIS, PROCEDE POUR LEUR PRODUCTION ET LEURS APPLICATIONS A LA PRODUCTION DE PROTEINES ETRANGERES OU DE PRINCIPES ACTIFS VACCINANTS**
REKOMBINANTE PROTEINE DES FILAMENTOESEN HAEMAGLUTININS VON BORDETELLA,IM BESONDEREN BORDETELLA PERTUSIS, HERSTELLUNGSVERFAHREN UND IHRE VERWENDUNG ZUR HERSTELLUNG VON FREMDPROTEINEN ODER VAKZINEN
RECOMBINANT PROTEINS OF FILAMENTOUS HAEMAGGLUTININ OF BORDETELLA, PARTICULARLY BORDETELLA PERTUSSIS, METHOD FOR PRODUCING SAME, AND USES THEREOF FOR PRODUCING FOREIGN PROTEINS OR VACCINES

(30) Priorité: 19.04.1994 FR 9404661
(43) Date de publication de la demande: 29.01.1997
(73) Titulaire: INSTITUT PASTEUR DE LILLE, 59019 Lille Cédex (FR); INSTITUT NATIONAL DE LA SANTE ET DE LA RECHERCHE MEDICALE (INSERM), 75654 Paris Cédex 13 (FR)
(72) Inventeur: LOCHT, Camille, F-59830 Wann-Ehain (FR); RENAULD, Geneviève, F-69007 Lyon (FR); CAPRON, André, F-59113 Chalempin (FR); RIVEAU, Gilles, F-59800 Lille (FR); MENOZZI, Franco, B-7022 Mons-Hyon (BE); JACOB-DUBUISSON, Françoise, F-59155 Faches-Thumesnil (FR)
(74) Mandataire: Gutmann, Ernest
(86) Numéro de dépôt international: FR9500512
(87) Numéro de publication internationale: WO95028486

(56) Documents cités:
- EP-A- 0 453 216
- WO-A-92/18624
- JOURNAL OF BACTERIOLOGY, vol. 171, no. 11, 1989 pages 6345-6348, MILLER J. F. ET AL. 'Analysis of Bordetella pertussis virulence gene regulation by use of transcriptional fusions in Escherichia coli'
- JOURNAL OF BACTERIOLOGY, vol. 174, no. 3, 1992 pages 970-979, MILLER J. F. ET AL. 'Constitutive sensory transduction mutations in the Bordetella pertussis bvgS gene'
- JOURNAL OF BACTERIOLOGY, vol. 173, no. 7, 1991 pages 2385-2392, ROY C.R. ET AL. 'Identification of Bordetella pertussis regulatory sequences required for transcriptional activation of the fhaB gene and autoregulation of the bvgAS operon'
- INFECTION AND IMMUNITY, vol. 58, no. 9, 1990 WASHINGTON US, pages 2895-2905, DELISSE-GATHOYE A.-M. ET AL. 'Cloning, partial sequence, expression, and antigenic analysis of the filamentous hemagglutinin gene of Bordetella pertussis' cité dans la demande
- INFECTION AND IMMUNITY, vol. 62, no. 3, Mars 1994 WASHINGTON US, pages 769-778, MENOZZI F. D. ET AL. 'Heparin-inhibitable lectin activity of the filamentous hemagglutinin adhesin of Bordetella pertussis' cité dans la demande
- MOLECULAR MICROBIOLOGY, vol. 11, no. 2, Janvier 1994 pages 337-347, WILLEMS R. J. L. ET AL. 'Mutational analysis of the Bordetella pertussis fim/fha gene cluster: identification of a gene with sequence similarities to haemolysin accessory genes involved in export of FHA' cité dans la demande
- JOURNAL OF BACTERIOLOGY, vol. 170, no. 7, 1988 pages 2904-2913, STIBITZ S. ET AL. 'Genetic analysis of a region of the Bordetella pertussis chromosome encoding filamentous hemagglutinin and the pleiotropic regulatory locus vir' cité dans la demande
- MOLECULAR MICROBIOLOGY, vol. 4, no. 5, 1990 pages 787-800, DOMENIGHINI M. ET AL. 'Genetic characterization of Bordetella pertussis filamentous haemagglutinin: a protein processed from an unusually large precursor'
- MOLECULAR MICROBIOLOGY, vol. 9, no. 4, 1993 pages 653-660, LOCHT C. ET AL. 'The filamentous haemagglutinin, a multifaceted adhesin produced by virulent Bordetella spp.' cité dans la demande

## Description

### OBJETS DE L'INVENTION

Grâce à la technologie du génie génétique, il est, en principe, désormais possible d'exprimer n'importe quel gène dans un organisme hétérologue afin de disposer d'une quantité illimitée d'une protéine donnée à des fins industrielles ou de recherche. Les micro-organismes sont très souvent utilisés comme hôtes pour l'expression hétérologue.

Paradoxalement, malgré les progrès considérables observés durant les vingt dernières années, un des problèmes qui ont freiné l'exploitation industrielle des protéines recombinantes est lié à la difficulté de purification de ces molécules qui se trouvent généralement concentrées dans l'organisme qui les synthétise. La purification des protéines recombinantes pourrait être considérablement simplifiée si celles-ci étaient sécrétées dans le milieu de culture. La manipulation génétique d'une micro-organisme pour qu'il sécrète une protéine recombinante nécessite la connaissance des mécanismes moléculaires qui régissent les voies métaboliques de sécrétion. Ces mécanismes sont particulièrement complexes chez les bactéries gram négatives, où toute protéine sécrétée doit traverser deux membranes lipidiques avant d'atteindre le milieu extracellulaire. Par conséquent, les bactéries gram négatives ne sécrètent que peu de protéines.

La sécrétion de protéines est plus simple chez les bactéries gram positives du fait que celles-ci ne possèdent qu'une seule membrane lipidique. Malheureusement, ces micro-organismes produisent généralement aussi des protéases extracellulaires, néfastes pour les protéines recombinantes. La construction de bactéries gram positives déficientes en protéases a par conséquent été un important domaine d'investigation. Cependant, cette tâche s'est avérée difficile, car ces micro-organismes sécrètent souvent de multiples protéases, et la délétion des gènes codant pour ces protéases diminue la viabilité des souches et, par conséquent, leur utilité pour l'expression des gènes hétérologues. Idéalement, il faudrait donc utiliser des bactéries gram négatives ne produisant pas ou peu de protéases extracellulaires et possédant un mécanisme de sécrétion très efficace.

L'invention met à double profit les capacités des Bordetella, et plus particulièrement de B.pertussis qui ne semble pas produire de produire de protéases extracellulaires et de l'aisance avec laquelle peuvent être isolées les hémagglutinines filamenteuses de celles des Bordetella qui en synthétisent pour, entre autre, remédier aux difficultés sus-mentionnées.

Bordetella pertussis, l'agent étiologique de la coqueluche, est une bactérie gram négative qui produit et sécrète plusieurs protéines de taille importante, dont la toxine coquelucheuse (environ 107 kDa) et l'hémagglutinine filamenteuse (Fha; environ 220 kDa). La Fha est le produit majeur de sécrétion, elle est facilement détectable par coloration au bleu de Coomassie après électrophorèse du surnageant de culture.

La Fha est une protéine de 220 kDa produite et sécrétée par B.pertussis. Elle est l'adhésine majeure et le produit de sécrétion majeur de cet organisme (pour revue voir Locht, C. Bertin, P., Menozzi, F.D., et Renauld, G. (1993) Mol. Microbiol. 9, 653-660). Le gène de structure de la Fha, appelé fhaB, a été cloné dans plusieurs laboratoires (Brown, D.R., et Parker, C.D. (1987) Infect. Immun., 55, 154-161; Relman, D.A., Domenighini, M., Tuomanen, E., Rappuoli, R., et Falkowo, S. (1989) Proc. Natl. Acad. Sci. U.S.A, 86, 2637-2641 ; Delisse-Gathoye, A.-M., Locht, C., Jacob, F., Raaschou-Nielsen, M., Heron, I., Ruelle, J.-L., DeWilde, M., et Cabezon, T. (1990) Infect. Immun. 58, 2895-2905) et code pour un précurseur d'environ 367 kDa (Delisse-Gathoye et al., 1990 ; Domenighini, M., Relman, D., Capiau, C. Falkow, S., Prugnola, A., Scarlato, V., et Rappuoli, R. (1990) Mol. Microbiol. 4, 787-800). La partie N-terminale de ce précurseur correspond à la partie mature de la Fha et la partie C-terminale est perdue lors de la maturation et/ou sécrétion de la protéine.

En aval du gène fhaB se trouve un opéron polycistronique responsable à la fois de la biogenèse de la Fha et des fimbriae, aussi appelées agglutinogènes (Locht, C. Geoffroy, M.C., et Renauld, G. (1992) EMBO J. 11, 3175-3183). Cet opéron contient quatre cistrons dont les produits des trois premiers sont homologues aux protéines accessoires et à l'adhésine de pili de plusieurs bactéries gram négatives (Locht et al., 1992) et impliqués dans la biogenèse des fimbriae de B.pertussis, et dont le produit du dernier est homologue à Sh1B et HpmB et est impliqué dans la biogenèse de la Fha (Willems, R.J.L., Geuijen, C., van der Heide, H.G.J., Renauld, G., Bertin, P. van den Akker, W.M.R., Locht, C., et Mooi, F.R. (1994) Mol. Microbiol. 11, 337-347).

Par ailleurs, la région N-terminale de la Fha est homologue aux régions N-terminales des hémolysines ShIA et HpmA de Serratia marcescens et Proteus mirabilis, respectivement (Delisse-Gathoye et al., 1990). Ces hémolysines sont sécrétés chez ces deux microorganismes et la sécrétion implique l'interaction du produit du gène shIB ou hpmB avec la région N-terminale de ShlA et HpmA, respectivement (Braun, V. ondraczed, R., et Hobbie, S. (1993) Zbl. Bakt. 278, 306-315). Des expériences de mutagénèse du gène fhaB ont montré que la région N-terminale de la Fha, homologue aux ShIA et HpmA, est égalemet importante pour la biogenèse de la Fha (Willems et al., 1994) suggérant donc par analogie avec les systèmes de sécrétion des hémolysines, que le produit du gène fhaC interagit avec la région N-terminale et que cette interaction est importante dans le processus de biogenèse de la Fha. Les protéines HpmB, ShIB et FhaC sont vraisemblablement des protéines de la membrane externe (Braun et al., 1993; Willems et al., 1994) et jouent un rôle dans la sécrétion des hémolysines et de la Fha, respectivement, au travers de la membrane externe. Chez B.pertussis, un blocage de la sécrétion au travers de la membrane externe conduit à la dégradation rapide de la protéine.

La Fha est une adhésine majeure de B.pertussis et exprime au moins trois types d'activités de liaison (voir Locht et al., 1993). Relman et al. (Relman, D., Tuomanen, E., Falkow, S., Golenbock, D., Saukkonen, K., et Wright, S. (1990) Cell 61, 1375-1382) ont montré qu'une séquence RGD dans la Fha mature est responsable de l'interaction de cette molécule avec l'intégrine CR3 (Â_{M}ß₂, CD11b/CD18) des macrophages. Cette interaction induit l'internalisation des B.pertussis dans les macrophages où ces organismes peuvent survivre.

La Fha peut également interagir avec des glycoconjugués et le domaine de reconnaissance des carbohydrates a été identifié par Prasad et al. (Prasad, S. M., Yin, Y., Rodzinski, E., Tuomanen, E. I., et Masure, R. (1993) Infect. Immun. 61, 2780-2785) dans la région 1141 à 1279 de la Fha, un peu en aval du site RGD. Menozzi et al. (Menozzi, F.D., Gantiez, C., et Locht, C. (1991) FEMS Microbiol. Lett. 78, 59-64) ont montré que la Fha exprime une affinité pour l'héparine et peut être purifiée par chromatographie sur héparine-sépharose à partir de surnageant de culture de B.pertussis. Cette interaction avec des glysosaminoglycans sulfatés semble jouer un rôle dans l'interaction des microorganismes avec les cellules épithéliales (Menozzi, F.D., Mutombo, R., Renauld, G., Gantiez, C., Hannah, J.H., Leininger, E., Brennan, M.J. et Locht, C. (1994) Infect. Immun. 62, 769-778).

La Fha est un bon immunogène pour l'induction d'lgAs dans les voies respiratoires chez le patient infecté par B.pertussis (Zackrisson, G., Lagergard, T., Trollfors, B., et Krants, I. (1990) J. Clin. Microbiol. 28, 1502-1505) et la présence d'IgAs est détectable encore longtemps après l'infection (Zackrisson, G., Arminjon, F., Krantz, I., Lagergard, T., Sigurs, N., Taranger, J. et Trollfors, B. (1988) Eur. J. Clin. Microbiol. Infect. Dis. 7, 764-770). Une réponse immune de longue durée contre la Fha peut également être observée chez la souris expérimentalement infectée avec B.pertussis par voie nasale (Amsbaugh, D.F., Li, Z.-M., et Shahin, R.D. (1993) Infect. Immun. 61, 1447-1452). Une bonne réponse immune (à la fois IgAs et IgG) contre la Fha peut aussi être obtenue dans les voies respiratoires de la souris après vaccination intra-nasale avec la Fha purifiée (Shahin, R.D., Amsbaugh, D.F., et Leef, M.F. (1992) Infect. Immun. 60, 1482-1488; Cahill, E.S., O'Hagan, D.T., Illum, L., et Redhead, K. (1993) FEMS Microbiol. Lett. 107, 211-216). Il est possible que l'une ou plusieurs des activités de liaison exprimées par la Fha soi(en)t responsable(s) de l'immunogénicité mucosale de cette molécule.

L'invention met à profit le mécanisme moléculaire de sécrétion de la Fha de Bordetella, notamment de B.pertussis pour la production de protéines ou peptides recombinants hétérologues de ces organismes.

Dans l'une de ces premières applications, l'invention permet, notamment dans les conditions gui seront décrites plus loin, la sécrétion de ces peptides recombinants hétérologues dans le milieu hétérologue de culture et, le cas échéant, la récupération de la partie hétérologue de ce peptide recombinant, lorsque celui-ci constitue le produit finalement recherché. Mais une autre variante de l'invention a pour objectif l'exposition du peptide recombinant à la surface de cellules procaryotes, notamment à des fins de vaccination.

La fusion de protéines ou peptides hétérologues avec la Fha peut, en effet avoir une application particulièrement intéressante dans le domaine de la vaccination. En effet, la Fha est capable de stimuler une réponse immunitaire mucosale importante et de longue durée après infection naturelle chez l'homme ou immunisation intranasale. Cette propriété est probablement due aux activités spécifiques d'attachement de la Fha aux muqueuses. Une fusion traductionnelle de la Fha avec un antigène pourrait donc faciliter la présentation de cet antigène au niveau des muqueuses nasales en vue de la production d'immunoglobulines sécrétoires (IgAs). Une telle stratégie est particulièrement intéressante pour une vaccination contre certaines maladies respiratoires et, lorsque le système immunitaire mucosal des voies respiratoires communique avec celui des autres muqueuses ou plus généralement d'autres cellules: cellules épithéliales, macrophages, etc..., ce principe peut s'élargir à de nombreuses autres maladies infectieuses contre lesquelles il est important de développer une immunité mucosale. Un tel type de vaccin peu onéreux pourrait facilement s'administrer par une vaporisation nasale. Cette voie de vaccination éliminerait donc le trauma causé par injection ainsi que le risque de destruction des vaccins oraux dans l'environnement acide de l'estomac.

Il est fait référence dans ce qui suit, aux dessins dont les légendes sont indiquées à la fin de cette description.

L'invention concerne tout d'abord l'ADN recombinant contenant une séquence (1) codant pour un potypeptide hétérologue vis-à-vis d'une Fha de B.pertussis fusionnée dans le même cadre de lecture à une séquence (2) placée en amont de la première, cette séquence (2) codant au moins pour la région N-terminale de la protéine mature de Fha qui, lorsque celle-ci est elle-même placée sous le contrôle d'un promoteur reconnu par les polymérases cellulaires de B.pertussis et introduite dans une culture de B.pertussis, est exprimée dans cette culture sous le contrôle de ce promoteur et excrétée dans le milieu de culture.

Dans l'un des cas extrêmes, la séquence (2) de l'ADN recombinant susdit code pour la totalité du précurseur de la Fha, par exemple celle de B.pertussis (parfois désignée sous l'abréviation FhaB). L'incorporation de cet ADN recombinant sous forme de plasmide notamment et sous le contrôle du promoteur adéquat dans une cellule B.pertussis conduit alors à l'expression de la protéine recombinante correspondante, dont une partie est excrétée complètement dans le milieu de culture, l'autre traversant également la membrane de B.pertussis, mais y restant attachée. Dans ce dernier cas, il sera vu dans ce qui suit, que la protéine recombinante, y incluse la séquence en acides aminés correspondantes aux polypeptides hétérologues se trouve exposée à la surface de ces cellules.

La protéine excrétée dans le milieu de culture peut être davantage purifiée, notamment par un procédé consistant à mettre en contact le milieu de culture avec l'héparine immobilisée sur un support insoluble pour former un complexe héparine-Fha, la protéine recombinante pouvant alors être récupérée par dissociation du complexe.

Dans la suite de cet exposé, il sera vu que les premiers essais ont été effectués dans une souche B.pertussis BGR4 dont la partie la plus importante de la phase de lecture du gène fhaB et son promoteur avaient été délétés du chromosome par deux éléments successifs de recombinaison homologues. L'ADN recombinant contenait un fragment EcoRI d'environ 10 kb isolé à partir d'un clone qui avait été entièrement séquencé, notamment par Delisse-Gathoye et al., 1990. C'est par rapport à la séquence décrite par ces auteurs que sont précisées dans le corps du présente texte, les positions relatives de certains des nucléotides dans le chromosome B.pertussis correspondant, le premier site d'EcoRI E^{a} correspondant à la position 1, et le second site E^{b} occupant alors la position 10035.

L'ATG d'initiation de la traduction se trouve en aval du site E^{a} (l'un des trois ATG aux positions respectivement 253, 298, 427) le promoteur correspondant étant intercalé entre le site E^{a} et l'ATG d'initiation pertinent. Le précurseur s'étend jusqu'au delà de la position du site E^{b} (position 11025).

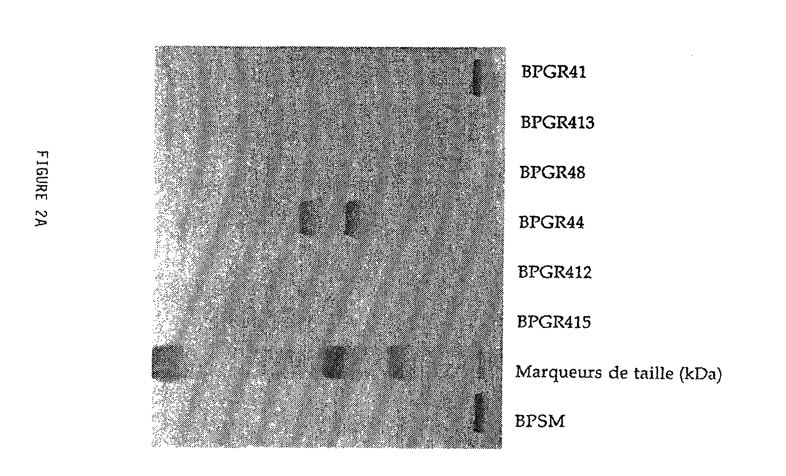

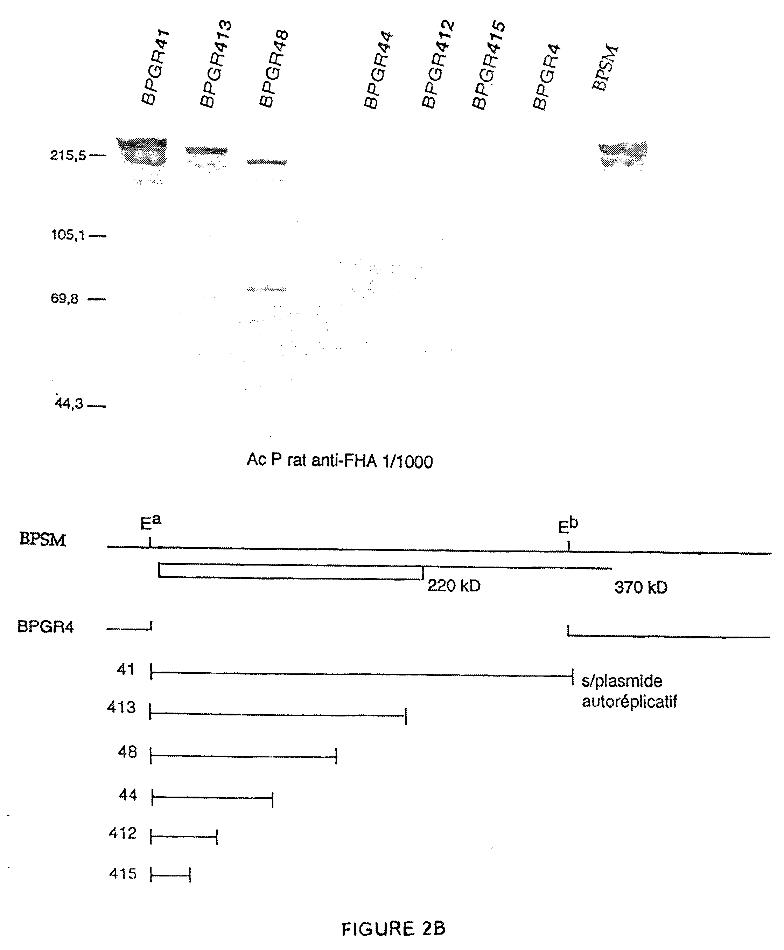

Comme cela sera décrit de façon plus détaillée dans les exemples, plusieurs ADNs recombinants ont été produits, lesquels comportaient notamment des séquences s'étendant toutes entre le nucléotides 1 et respectivement le nucléotide 10035 (BPGR41), 6292 (BPGR413), 5215 (BPGR48), 2841 (BPGR44), 1575 (BPGR412) et enfin 907 (BPGR415). Des sites de restriction correspondants sont indiqués dans la **figure 2B**.

Les observations qui ont été faites quant à l'expression de ces fragments de tailles décroissantes, avant que ceux-ci n'aient été recombinés avec une séquence codant pour un polypeptide hétérologue, étaient les suivantes. Comme il résulte des **figures 2A et 2B,** on obtenait une excrétion importante du peptide codé par le peptide BPGR41, excrétion qui allait se réduire pour les fragments contenus dans les plasmides BPGR413 et BPGR48, qui ne contenaient plus que la séquence codant pour la presque totalité de la protéine mature (BPGR413) et une séquence tronquée codant pour un polypeptide également tronqué (BPGR48). Ces observations se trouvent reflétées par les essais illustrés dans la **figure 2B** dans lesquels les produits d'expression ont été détectés par des anticorps polyclonaux de rats anti-Fha. Mais dans ce test, on constate l'absence d'expression détectable pour les plasmides contenant des séquences plus courtes. L'on constate au contraire dans un autre système de mesure (coloration au bleu de Comassie : **figure 2A**) une recrudescence de l'expression avec le plasmide BPGR44. Sans qu'il y ait à y voir une relation nécessaire de correspondance, on constate que la partie reconnue par la majeure partie des anticorps polyclonaux de la séquence Fha n'est pas nécessaire à la production d'une excrétion dans le système qui a été utilisé. Lorsqu'est raccourcie davantage la séquence tronquée de Fha, on constate de nouveau une réduction de l'excrétion. Ainsi, le fragment contenu dans le plasmide BPGR412 est-il encore exprimé à un moindre degré, même si aucune bande n'est observée sur la figure 2 pour la piste d'électrophorèse sur gel correspondante au plasmide BPGR412. Mais, la mise en contact d'une culture correspondante avec l'héparine immobilisée a permis d'isoler une fraction excrétée reconnue par des anticorps monoclonaux reconnaissant spécifiquement la région N-terminale de la Fha.

Il apparaît que des séquences (2) entrant dans les ADNs recombinants selon l'invention doivent, en tout état de cause, comporter des signaux d'excrétion de la séquence codant pour la Fha et la région N-terminale homologue aux régions N-terminales des hémolysines Sh1A et HpmA de Serratia marcescens et Proteus mirabilis.

Pour la mise en oeuvre de l'une des applications préférées de l'invention, à savoir la production d'un peptide hétérologue et sa récupération à partir du milieu de culture, il apparaît par conséquent que l'extension de la séquence (2) à partir de l'extrémité N-terminale de la Fha vers son extrémité C-terminale sera choisie de façon à ne pas aller au delà de la longueur qui ferait que la transformation de B.pertussis avec cet ADN recombinant alors placé sous le contrôle d'un promoteur susceptible d'être reconnu par B.pertussis ne permettrait plus l'excrétion directe de la protéine recombinante alors formée dans le milieu de culture de ce B.pertussis.

Dans le cadre de cette application, un ADN recombinant préféré est caractérisé en ce que la séquence (2) s'étend entre l'ATG correspondant au codon d'initiation de la traduction de la Fha à un nucléotide C-terminal au delà du nucléotide 907 dans la direction de la traduction et de préférence non au delà de la position 6292.

Sans que ce test soit décisif, il peut encore être affirmé qu'un ADN recombinant préféré sera celui qui est caractérisé par le fait qu'il ne réagit plus que faiblement avec des anticorps anti-Fha plus particulièrement dirigés contre les épitopes de la partie C-terminale de la Fha mature, situés au delà du site nucléotidique 2841 dans le sens de la traduction.

Il va de soi que la séquence recombinante reprenant les séquences (1) à et (2) peut être réalisée de toute façon en soi connue, en fonction du but final que l'on se sera donné. Eventuellement, la séquence (1) codant pour le peptide hétérologue se trouvera encadrée par des régions courtes codant pour des peptides pré-définis formant des sites de clivage spécifiques d'enzymes protéolytiques elles-mêmes spécifiques, ce grâce à quoi la partie hétérologue du polypeptide recombinant peut aisément être séparée à partir de celui-ci.

Dans une autre application avantageuse de l'invention, le peptide recombinant est susceptible d'être utilisé comme principe vaccinant, la séquence peptidique hétérologue étant douée des propriétés immunogènes choisies au préalable et, de préférence, la partie dérivée de la protéine mature de Fha comportant l'un au moins des sites spécifiques d'attachement de la Fha à des muqueuses ou de façon plus générale à d'autres cellules eucaryotes, notamment à des cellules épithéliales ou des macrophages.

L'utilisation de l'ADN recombinant pour la production de polypeptide hétérologue peut être envisagée dans des cellules procaryotes autres que Bordetella pertussis ou même que plus généralement des Bordetella. Il est en effet rappelé que Stibitz, Weiss et Falkow ont rapporté des ADNs de Bordetella contenant la séquence codant pour le précurseur de la Fha et l'ensemble des gènes de régulation, y inclus le gène fhaC, c'est-à-dire le gène auxiliaire dont le produit d'expression est également nécessaire à l'expression de la Fha, pouvaient, quand ils étaient transportés dans E.coli d'être exprimés et exposés à la surface des bactéries E.coli transformées (J.of Bacteriology (1988) 170, 2904-2913).

Il va de soi que l'invention concerne donc toutes les cultures cellulaires dans lesquelles la Fha peut être exprimée. L'invention concerne donc plus particulièrement les cultures de cellules appartenant à une espèce Bordetella, notamment B.pertussis, dès lors que ces cellules sont également porteuses du gène fhaC exprimable dans ces cellules.

L'invention concerne encore les cultures cellulaires transformées, appartenant à des espèces autres que Bordetella, dès lors qu'elles contiendraient également une séquence codant pour au moins la partie de la FhaC nécessaire à l'expression de la séquence (2) sous une forme également exprimable au sein des cellules de cette culture.

Il en est ainsi de E.coli, et, le cas échéant, sous réserve d'adaptation mineures autorisant l'expression des ADNs recombinants de l'invention dans d'autres bactéries gram négatives, par exemple du type salmonella, Vibrio, etc...

Il est rappelé que Willems et al. (1994) Mol. Microbiol. 11, 337-347 ont séquencé entièrement une séquence codant pour la protéine FhaC.

Il va également de soi que l'invention n'est pas limitée à des ADNs recombinants contenant une séquence codant pour la Fha de B.pertussis. Celle-ci peut être remplacée par toute séquence correspondante isolable à partir d'autres Bordetella, qu'il s'agisse de Bordetella infectieuses pour l'homme, notamment B.parapertussis ou B.bronchiseptica, ou encore pour des Bordetella infectieuses pour des animaux, notamment les Bordetella bronchiseptica infectieux pour le chien ou le porc.

L'invention n'est nullement limitée aux ADNs recombinants dont les séquences (2) sont restreintes à des séquences tronquées de l'ADN codant pour une Fha de Bordetella. Comme cela a été vu plus haut, l'invention concerne également les ADNs recombinants contenant des séquences (2) plus longues, chaque fois que sera au contraire recherchée la production de cellules procaryotes, notamment de bactéries portant, exposé à leur surface, le produit d'expression de l'ADN recombinant susdéfini. La séquence hétérologue (1) peut soit être incorporée à l'intérieur même de la séquence codant pour Fha, voire même FhaB, soit être fusionnée à la Fha mature ou le précurseur avec conservation du code de lecture correspondant.

Dans le cas où la cellule hôte, le cas échéant, après atténuation ou inactivation, peut être utilisée comme support de vaccin, on apprécie que l'invention fournit de nouvelles variétés vaccinantes comportant des cellules procaryotes de ce type portant exposées à leur surface le produit d'expression de l'ADN recombinant. Avantageusement, seront exposés à la surface des bactéries en cause, à la fois la séquence en acides aminés correspondante aux sites antigéniques du peptide hétérologue, d'une part, et l'un des sites d'adhésion de la protéine Fha aux muqueuses ou encore à d'autres cellules eucaryotes, telles que des cellules épithéliales ou des macrophages.

A titre d'exemples de technique pouvant être utilisés pour obtenir cette orientation convenable, on fera référence au brevet européen n°0242243 déposé le 06/03/87.

Alors que dans le cas de la production in vitro d'une protéine ou d'une polypeptide recombinant, l'on pouvait avoir des cellules procaryotes transformées par un plasmide, il apparaît que pour la réalisation de bactéries porteuses du produit d'expression de l'ADN recombinant à leur surface, il est préférable que celui-ci ait été incorporé sous le contrôle du promoteur approprié à l'ADN chromosomique desdites cellules. Toutes techniques connues à cet effet peuvent être mises en oeuvre, telles que les techniques de recombinaison homologue.

Il va de soi que la séquence (1) peut coder pour toutes séquences antigéniques souhaitées, qu'il s'agisse d'antigènes de Bordetella, de Shigella, de Neisseria, de Borrellia, etc... de toxines ou toxoïdes diphtériques, tétaniques ou colériques, d'antigènes viraux, notamment de l'hépatite B, de l'hépatite C, du polyovirus ou de HIV, d'antigènes parasitaires tels que des plasmodium, des Shistozoma, des toxoplasmes, etc... Il s'agit là évidemment d'exemples qui n'ont aucun caractère limitatif.

De même, les hôtes cellulaires peuvent être constitués par toutes bactéries atténuées ou inactivées, telles que des shigelles atténuées, des E.coli atténués ou des salmonelles atténuées.

Mais, on l'a vu, tout fha de Bordetella peut être mis en oeuvre. Partant d'une séquence codant pour l'une d'entre elles, on sait que l'on peut détecter des séquences correspondantes contenues dans l'ADN chromosomiques d'autres Bordetella, par exemple à l'aide de sondes appropriées.

L'invention présente un intérêt tout particulier pour la constitution de compositions immunogènes et vaccinantes, destinées à l'administration par mise en contact de muqueuses, notamment pour l'administration par voies nasales. L'invention est donc d'un intérêt tout particulier pour la prévention d'infection des voies respiratoires ou de tissus susceptibles d'être atteints par ces voies. Les compositions du genre en question peuvent se présenter sous forme d'aérosol, administrables notamment par voies nasales. L'invention s'intéresse à la vaccination aussi bien humaine que vétérinaire.

D'autres caractéristiques de l'invention apparaîtront encore au cours de la description qui suit, des constructions et essais biologiques qui ont été effectués dans le cadre de l'invention et qui lui apporte le soutien expérimental requis.

### FIGURES

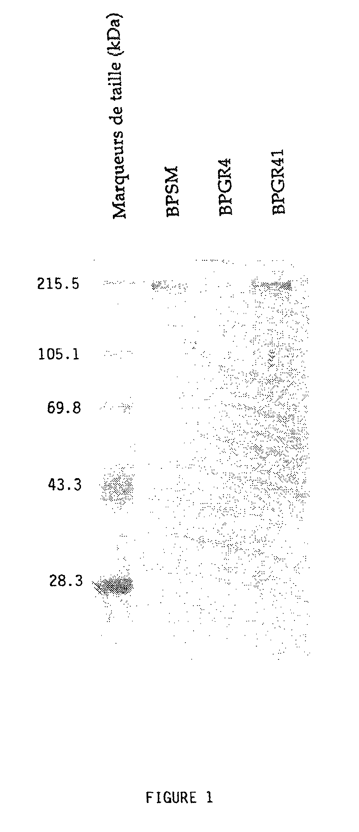

**Figure 1.** Analyse par SDS-PAGE et coloration au Bleu de Coomassie des surnageants de culture des souches *B. pertussis* BPSM, BPGR4 et BPGR41. Les marqueurs de taille et les surnageants de culture non concentrés des trois souches de *B. pertussis* ont été déposés sur gel de SDS-polyacrylamide. Après électrophorèse, le gel a été coloré au Bleu de Coomassie comme décrit par Sambrook *et al*. (1989). Les poids moléculaires des marqueurs de taille sont montrés dans la marge.
**Figures 2A et 2B.** Analyse par électrophorèse (A) et Western blot (B) des sumageants de culture des souches *B. pertussis* BPGR41, BPGR413, BPGR48, BPGR44, BPGR412, BPGR415, BPGR4 et BPSM. Après électrophorèse, le gel a été coloré au Bleu de Coomassie comme décrit pour la figure 1 (A) ou transféré sur membrane de nitrocellulose et analysé par Western blot comme décrit par Delisse-Gathoye *et al*. (1990) à l'aide d'un anticorps polyclonal anti-FHA de rat à une dilution de 1/1000. La carte des constructions qui sont à la base des différentes souches est montrée. E^{a} et E^{b} représentent respectivement le premier et deuxième site *Eco*RI du gène *fhaB* (Delisse-Gathoye *et al*., 1990).

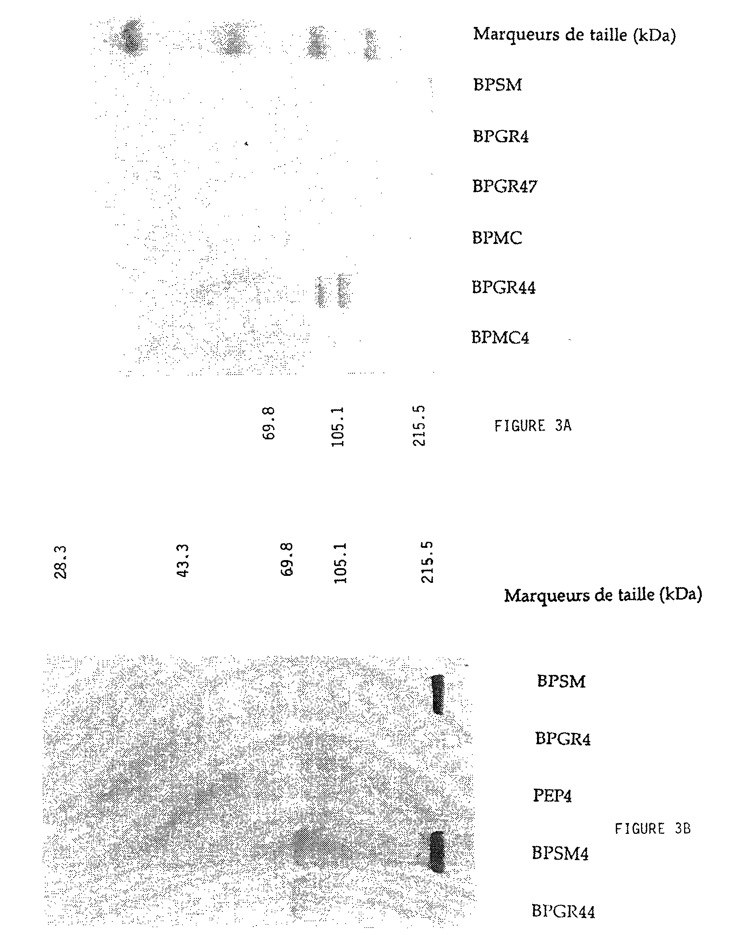

**Figures 3A et 3B.** Analyse par électrophorèse (A) et Western blot (B) des surnageants de culture des souches *B. pertussis* BPSM, BPGR4, BPMC, BPGR44, BPGM47, BPMC4, BPSM4 et *B. parapertussis* PEP4. Après électrophorèse, le gel a été coloré au Bleu de Coomassie comme décrit pour la figure 1 (A) ou analysé par Western blol en utilisant l'antisérum anti-FHA de rat à une dilution de 1/500 (B) comme décrit pour la figure 2.

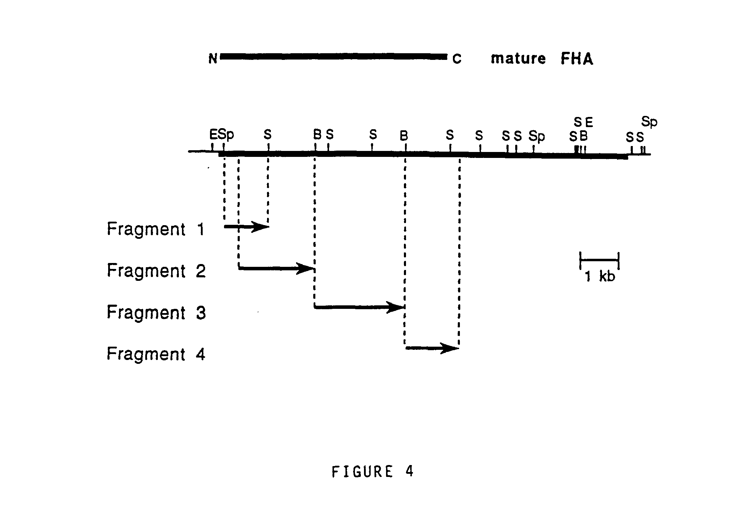

**Figure 4.** Carte de restriction des différents fragments d'ADN exprimé en fusion avec le gène codant pour MalE. La ligne noire en haut montre la longueur de la portion de *fhaB* qui code pour la FHA mature. N et C désignent respectivement les régions amino- et carboxy-terminales. La ligne noire au milieu montre la longueur de la phase ouverte de lecture de *fhaB*. E, *Eco*RI; Sp, *Sph*I; S, *Sal*I; B, *Bam*HI. Les flèches montrent la longueur des fragments exprimés et leur direction d'expression.

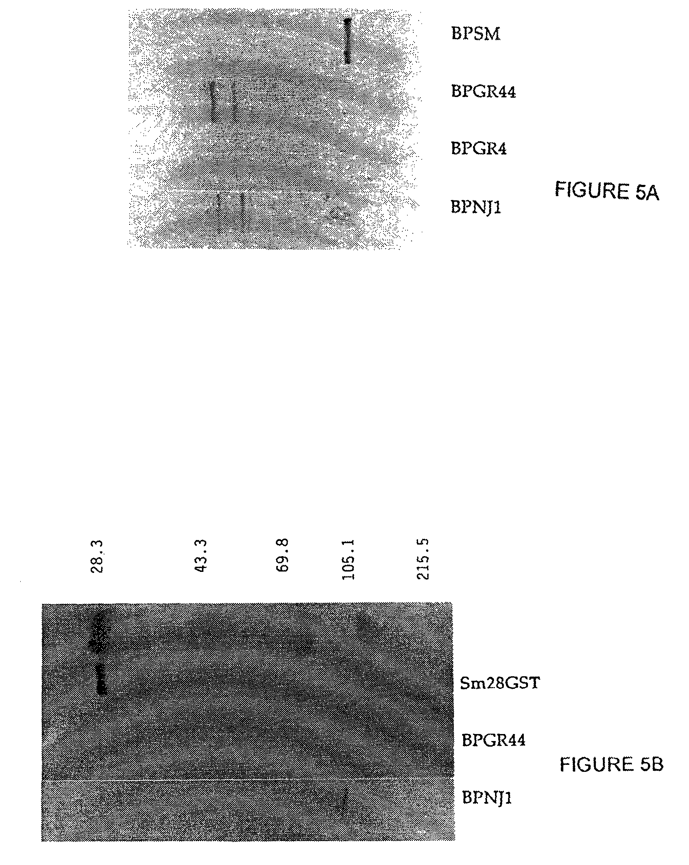

**Figures 5A et 5B.** Analyse Western blot des sumageants de culture des souches B. *pertussis* BPSM, BPGR44, BPGR4 et BPJN1. Après électrophorèse, les gels ont été analysés par Western blot comme décrit pour la figure 2 en utilisant l'antisérum anti-FHA de rat à une dilution de 1/500 (A) ou un anticorps anti-peptide 190-211 de Sm28GST de rat à une dilution de 1/250 (B). La piste de gauche du gel (B) contient la Sm28GST recombinante purifiée.

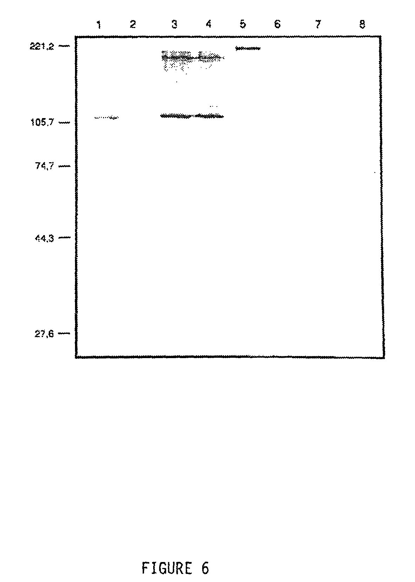

**Figure 6.** Analyse par Western blot des surnageants de culture et des protéines associées aux cellules des souches *B. pertussis* BPSM, BPGR4, BPGR5 et BPGR6. L'analyse par Western blot des protéines issues des surnageants de culture (pistes 5 à 8) ou de fractions protéiques associées aux cellules (pistes 1 à 4) des différentes souches de *B. pertussis* BPSM (pistes 1 et 5), BPGR4 (pistes 2 et 6), BPGR5 (pistes 3 et 7) et BPGR6 (pistes 4 et 8) a été réalisée comme décrit pour la figure 2 en utilisant l'anticorps monoconal anti-FHA 12.1.9 (Delisse-Gathoye *et al*., 1990).

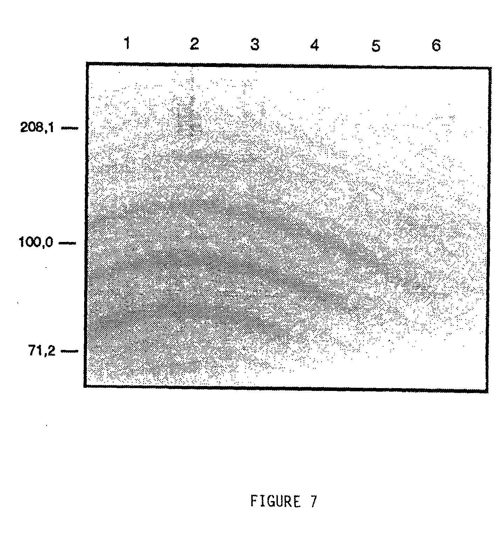

**Figure 7.** Analyse par Western blot des surnageants de culture et des protéines associées aux cellules des souches *B. pertussis* BPSM, BPGR5 et BPGR6. L'analyse par Western blot des protéines issues des sumageants de culture et purifiées sur matrice d'héparine-sépharose (pistes 4 à 6) ou de fractions protéiques associées aux cellules (pistes 1 à 3) des différentes souches de *B. pertussis* BPSM (pistes 1 et 4), BPGR5 (pistes 2 et 5) et BPGR6 (pistes 3 et 6) a été réalisée comme décrit pour la figure 2 en utilisant un antisérum anti-Sm28GST de lapin dilué 200 fois.

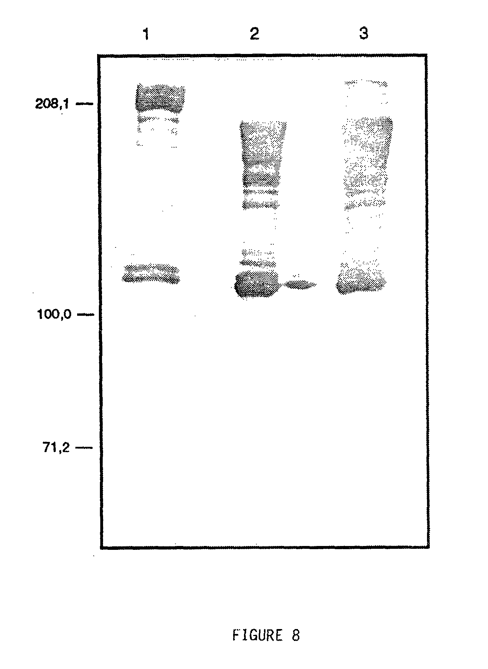

**Figure 8.** Analyse par Western blot des surnageants de culture purifiés sur colonne de héparine-sépharose des souches *B. pertussis* BPSM, BPGR5 et BPGR6. L'analyse par Western blot des protéines issues des surnageants de culture et purifiées sur matrice d'héparine-sépharose des souches de *B. pertussis* BPSM (piste 1), BPGR5 (piste 2) et BPGR6 (piste 3) a été réalisée comme décrit pour la figure 2 en utilisant l'anticorps monoclonal anti-FHA 12.1.9 (Delisse-Gathoye *et al*., 1990).

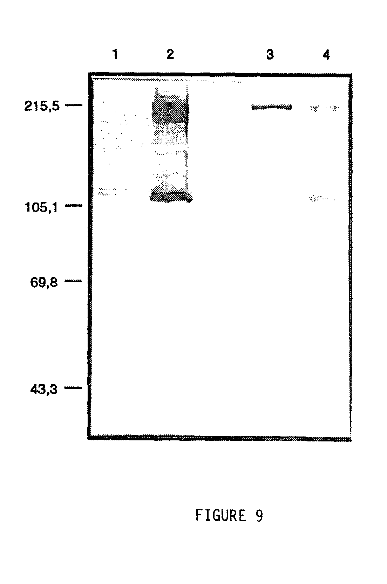

**Figure 9.** Analyse par Western blot des surnageants de culture et des protéines associées aux cellules des souches *B. pertussis* BPSM et BPGR60. L'analyse par Western blot des protéines issues des surnageants de culture (pistes 1 et 3) ou de fractions protéiques associées aux cellules (pistes 2 et 4) des souches *B. pertussis* BPSM (pistes 3 et 4), BPGR60 (pistes 1 et 2) a été réalisée comme décrit pour la figure 2 en utilisant l'anticorps monoclonal anti-FHA 12.1.9 (Delisse-Gathoye *et al*., 1990).

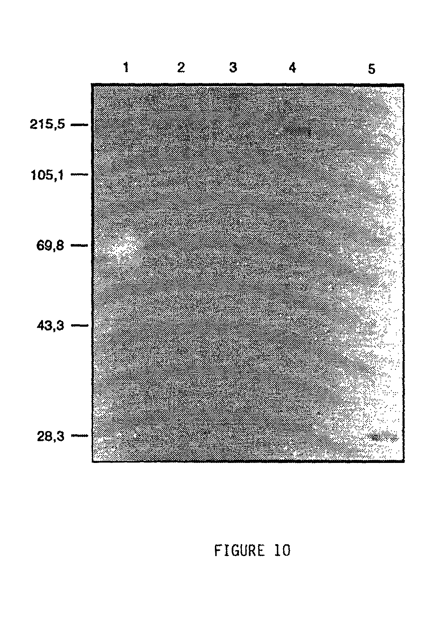

**Figure 10.** Analyse par Western blot des surnageants de culture et des protéines associées aux cellules des souches *B. pertussis* BPSM et BPGR60. L'analyse par Western blot des protéines issues des surnageants de culture (pistes 1 et 3) ou de fractions protéiques associées aux cellules (pistes 2 et 4) des souches *B. pertussis* BPSM (pistes 1 et 2), BPGR60 (pistes 3 et 4) a été réalisée comme décrit pour la figure 2 en utilisant un antisérum anti-Sm28GST de lapin. La piste 5 contient la Sm28GST recombinante purifiée.

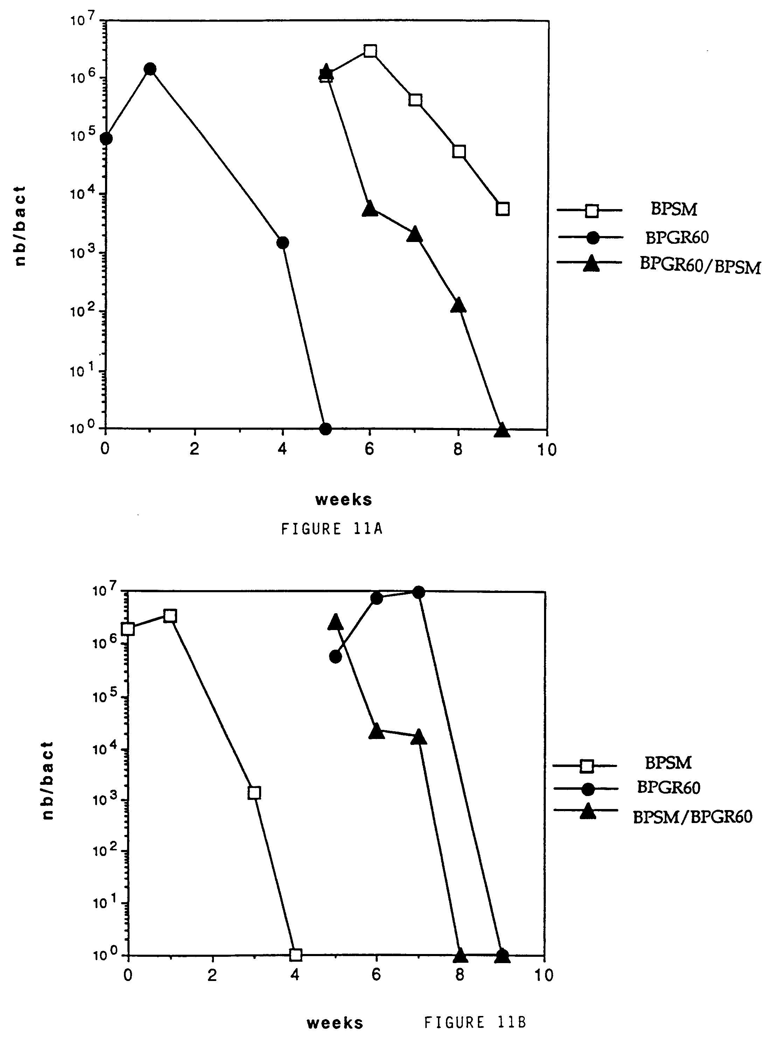

**Figures 11A et 11B.** Colonisation de souris OF1 par B. *pertussis* BPGR60 et Tohama I. Les souris OF1 ont été infectées par voie nasale avec les souches *B. perrussis* Tohama I (carrés blancs), BPGR60 (ronds noirs), BPGR60 et puis Tohama I (triangles noirs en A) ou Tohama I et puis BPGR60 (triangles noirs en B). Trois heures après l'infection, une série de souris a été sacrifiée et le nombre de B.pertussis viables estimé par poumon. Les autres groupes de souris ont été analysés une ou plusieurs semaines après infection comme montré sur la figure.

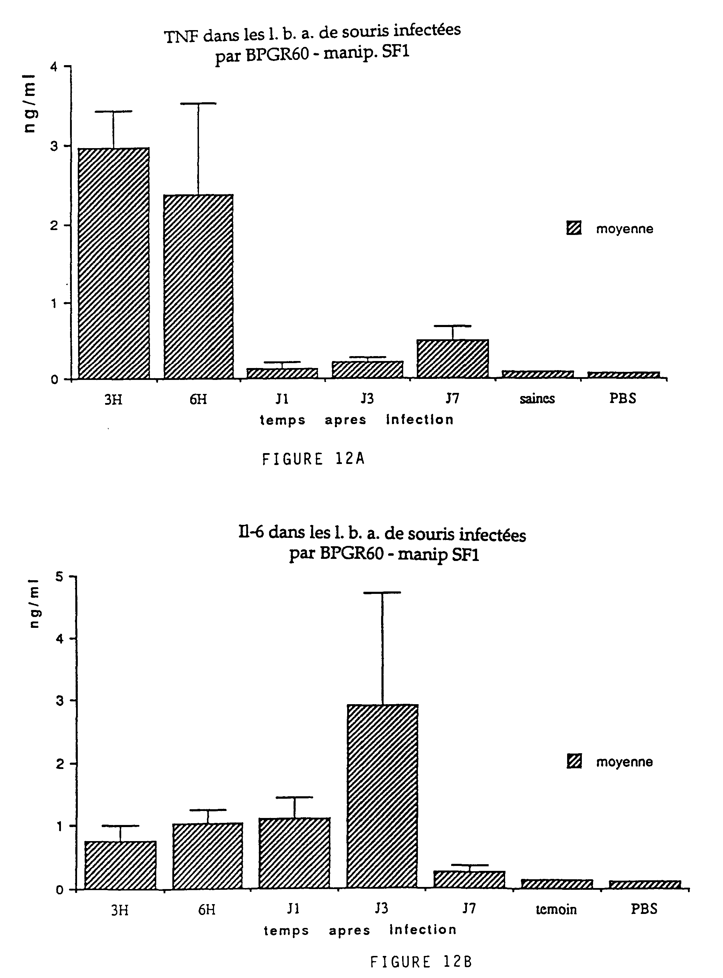

**Figures 12A et 12B.** Dosage de TNF et 11-6 des souris infectées par B.pertussis BPGR60. TNF (A) et II-6 (B) ont été dosées chez les souris non-infectées (saines) ou infectées par B.pertussis BPGR60 3 hrs, 6 hrs, 1 jour, 3 jours ou 7 jours après infection.

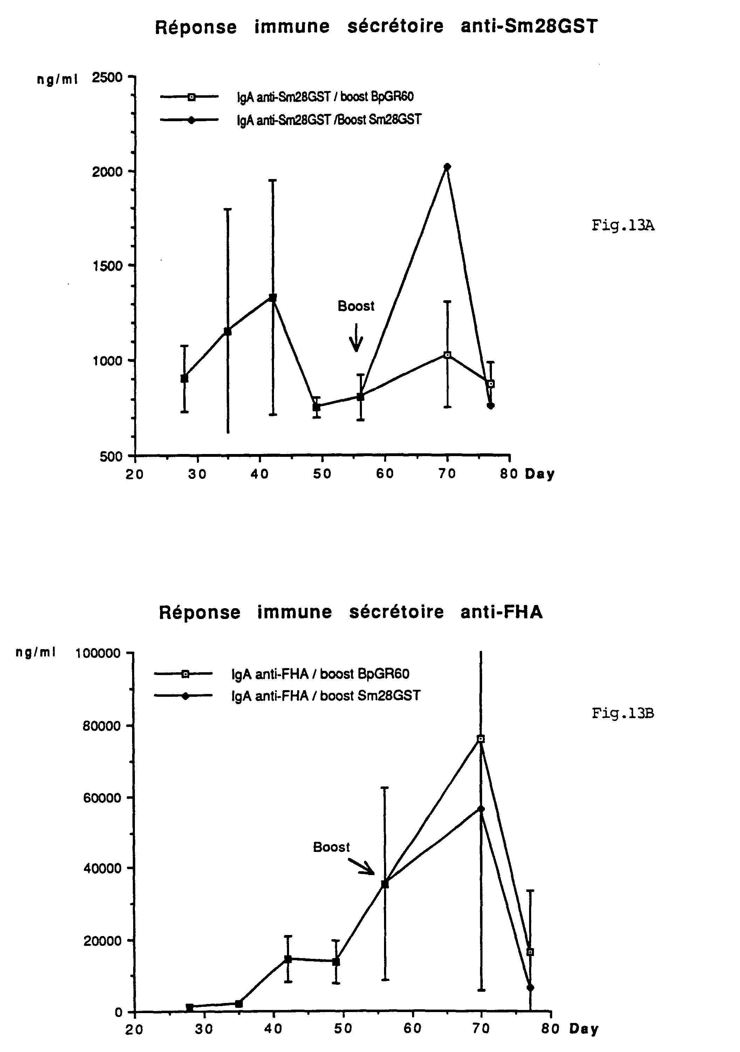

**Figures 13A et 13B.** Dosage d'IgA anti-Sm28GST et anti-Fha dans les lavages bronchoalvéolaires des souris OF1 infectées par B.pertussis BPGR60. Les souris OF1 ont été infectées avec B.pertussis BPGR60 par voie nasale. Après infection, aux jours indiqués sur la figure, des groupes de souris ont été sacrifiés et les IgA anti-Sm28GST (A) et anti-Fha (B) de leur liquide bronchoalvéolaires ont été dosés. Au jour 56 (en A) ou 63 (en B), 20 µg de Sm28GST (triangles noirs) ou une nouvelle dose de B.pertussis BPGR60 (carrés noirs) ont été administrés par voie nasale.

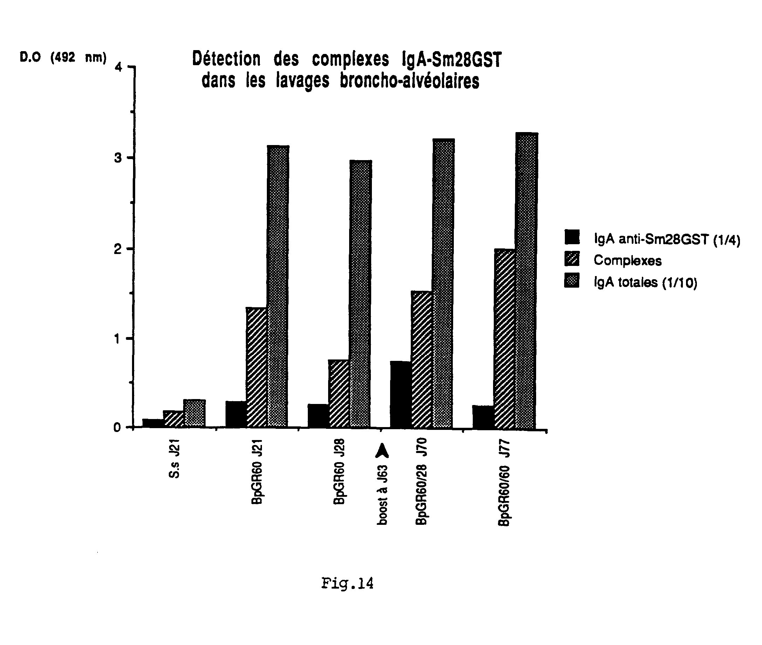

**Figure 14.** Dosage de complexes IgA-Sm28GST dans les lavages bronchoalvéolaires des souris traitées comme précédemment décrit dans la légende de la figure 13. La quantité de complexes (colonnes rayées) est présentée en comparaison avec les IgA libres anti-Sm28GST (colonnes noires), et les IgA totales (colonnes grises).

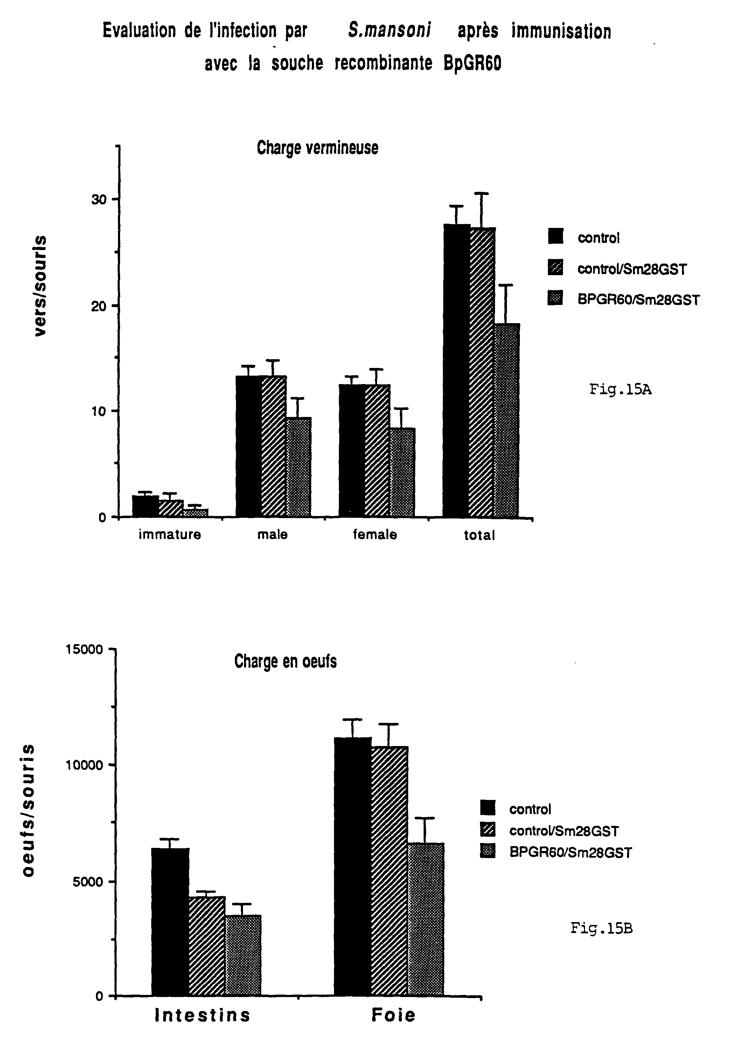

**Figures 15A et 15B.** Charge parasitaire observée après infestation par S.mansoni de souris OF1 préalablement immunisées par BPGR60 et rappelées par la Sm28GST libre. Les doses administrées sont identiques à celles utilisées pour les expériences d'immunisation (Fig. 13). Après 42 jours, les souris sont sacrifiées et le foie perfusé pour évaluation de la charge vermineuse (A). Le foie et les intestins sont chimiquement solubilisés et les oeufs tissulaires comptés (B). Colonne noire, souris non traitées ; colonne rayée, souris n'ayant reçu que la Sm28GST libre à J63, colonne grise, souris traitées par BPGR60 (J0) et par Sm28GST libre (J63).

### EXEMPLES

### I. Complémentation de la souche B. pertussis BPGR4 par un plasmide dérivé de pBBR1 contenant le gène fhaB.

Afin de savoir s'il est possible de complémenter une mutation chromosomique du gène *fhaB* par un plasmide autoréplicatif, nous avons utilisé la souche *B. pertussis* BPGR4 (Locht *et al*., 1992), une souche dérivée de la souche sauvage *B. pertussis* Tohama I chez laquelle le fragment *Eco*RI de 10 kb contenant la plupart de la phase de lecture du gène *fhaB* et son promoteur a été délété du chromosome par deux événements successifs de recombinaison homologue. Cette souche ne produit pas de FHA. D'autre part. le fragment *Eco*RI de 10 kb isolé de pRIT13202 (Delisse-Gathoye *et al*., 1990) et contenant la plupart du gène *fhaB* a été cloné dans le site *Eco*RI du plasmide pBBR122. Ce plasmide est un dérivé de pBBR1 isolé de *Bordetella bronchiseptica* et décrit par Antoine et Locht (Antoine, R., et Locht, C. (1992) *Mol. Microbiol*. **6,** 1785-1799). Il contient un fragment *Hha*I de 1364 pb provenant de pBR328 (Soberon, X., Covarrubias, L., et Bolivar, F. (1980) *Gène* **9**, 287-305) et conférant la résistance au chloramphénicol inséré dans le site *Pvu*I, ainsi que le gène commercial (Pharmacia) conférant la résistance à la kanamycine inséré au site *Ava*I en position 1388.

La digestion de pBBR122 par *Eco*RI et l'insertion du gène *fhaB* sous forme du fragment *Eco*RI de 10 kb inactive le gène de résistance au chloramphénicol mais maintient la résistance à la kanamycine. Le plasmide recombinant a été appelé pBG1 et a été introduit dans *B. pertussis* BPGR4 par électroporation. Cette nouvelle souche de *B. pertussis* est appelée *B. pertussis* BPGR41. L'analyse des surnageants de culture de *B. pertussis* BPSM, un dérivé Sm^{R} de la souche Tohama I (Menozzi *et al*., 1994), de *B. pertussis* BPGR4 et de *B. pertussis* BPGR41 par electrophorèse en SDS-polyacrylamide (SDS-PAGE) et coloration au bleu de Coomassie (Fig. 1) ainsi que par Western blot en utilisant des anticorps polyoclonaux de rat spécifiques à la FHA montrent que pBG1 peut efficacement complémenter la mutation *fhaB* de *B. pertussis* BPGR4.

### 11. Déletions progressives de la région C-terminale de FhaB.

Le produit primaire du gène *fhaB*, *i. e*. le précurseur de la FHA, est appelé FhaB. Puisque la région N-terminale homologue aux hémolysines ShlA et HpmA est importante pour la biogenèse de la FHA (Willems *et al*., 1994), il était important d'investiguer le rôle de la région C-terminale de FhaB dans la biogenèse de la FHA. Plusieurs délétions de la région C-terminale ont été obtenues : pBG13 est le résultat de l'échange du fragment *Sph*I/*Bam*HI de 2.5 kb de pBG4 par le fragment *Sph*I/*Bgl*II de 6 kb de pRIT13202 (Delisse-Galhoye *et al*., 1990); pBG8 est le résultat de l'insertion du fragment *Bam*HI de 4.7 kb dans pBG4 digiré par *Bam*HI; pBG4 est le résultat de la digestion de pBG1 par *Bam*HI et de sa religation, ce plasmide a donc perdu les deux fragments *Bam*HI de 4.7 kb et de 2.37 kb; pBG12 est le résultat de l'échange du fragment *Sph*I/*Bam*HI de 2.5 kb de pBG4 par le fragment *Sph*I/*Bam*HI de 1.27 kb de pUC18-3, pUC18-3 a été généré en échangeant le fragment *Sph*I/*Sal*I de 15 pb de pUC18 par le fragment *Sph*I/*Sal*I de 1.27 kb de pRIT13197 (Delisse-Gathoye et al., 1990); pBG15 est le résultat de la religation des deux fragments *Pvu*I de 3.65 kb et 2.76 kb après digestion de pBG4 par PvuI, générant ainsi la délétion du fragment *Pvu*I de 1.9 kb. Les plasmides pBG13, pBG8, pBG4, pBG12 et pBG15 ont été introduits dans *B. pertussis* BPGR4 par électroporation ce qui a généré les souches *B. pertussis* BPGR413, BPGR48, BPGR44, BPGR412 et BPGR415, respectivement.

Les surnageants de culture de ces différentes souches ont été analysés par SDS-PAGE et coloration au bleu de Coomassie ainsi que par Western blot en utilisant des anticorps polyclonaux de rat anti-FHA. Les résultats sont montrés dans la figure 2 et indiquent que par rapport à la souche *B. pertussis* BPSM et la souche *B. pertussis* BPGR41, les souches BPGR413 et BPGR48 produisent beaucoup moins de FHA dans le surnageant de culture. Par contre, la souche BPGR44 produit plus de FHA tronquée et sécrétée que la souche BPSM ou la souche BPGR41. Les souches BPGR412 et BPGR415 produisent à nouveau moins de FHA tronquée que la souche BPGR44, bien due la FHA tronquée produite par BPGR412 est clairement visible dans le surnageant de cutlure. Ces expériences montrent l'importance de la région C-terminale de FhaB dans la biogenèse et/ou sécrétion de la FHA mature, mais ils montrent aussi qu'une FHA tronquée (par exemple chez la souche BPGR44) peut être très efficacement sécrétée en absence de la région C-terminale de FhaB.

### III. Importance de fhaC dans la biogenèse de la FHA tronquée codée par pBG4 et sécrétion chez Bordetella parapertussis

Puisque *B. pertussis* BPGR44 sécrète très efficacement la FHA tronquée, il était important de savoir si cette sécrétion était toujours dépendante du produit du gène *fhaC.* pBG4 a donc été introduit dans la souche *B. pertussis* BPMC (Locht et al., 1992). Cette souche est caractérisée par une délétion chromosomique du gène *fhaB* total et de la région intergénique entre *fhaB* et les gènes auxiliaires en aval, y compris *fhaC*. Elle n'exprime donc ni *fhaB*, ni *fhaC*, ni les gènes *fimBCD* (aussi appelés *fhaDAE*). Les analyses par SDS-PAGE/coloration au bleu de Coomassie des surnageants de culture de la souche *B. pertussis* BPMC (pBG4), appelée BPMC4, montrent que cette souche ne produit pas de FHA tronquée extracellulaire. Ces résultats (Fig. 3) montrent donc que l'expression du gène *fhaC* est nécessaire à la production extracellulaire de la région N-terminale de la FHA. L'importance de la région N-terminale de la FHA homologue aux hémolysines ShlA et HpmA (Delisse-Gathoye *et al*., 1990) pour la sécrétion de la FHA tronquée a été étudiée en générant la souche *B. pertussis* BPGR47. Cette souche est un dérivé de BPGR4 transformé avec pBG7. Ce plasmide est le résultat de l'échange du fragment *Sph*I/*Bam*HI de 2.5 kb de pBG4 par le fragment *Sph*I/*Bam*HI de pUC18-5, qui est lui-même le résultat de l'insertion du fragment *Sal*I/*Bam*HI d'environ 1.27 kb du pRIT13120 (Delisse-Gathoye et al., 1990) dans pUC18-4 digéré par *Sal*I et *Bam*HI. Le plasmide pUC18-4 est le fruit de la digestion de pUC18-3 par *Pst*I et de la religation sur lui-même. Cette construction délète en phase un fragment *Pst*I d'environ 460 pb codant pour la région de la FHA qui est homologue aux hémolysines ShIA et HpmA. L'analyse de la souche BPGR47 par électrophorèse et Western blot (Fig. 3) montre que la région homologue est également nécessaire à la sécrétion de la FHA tronqué, comme à celle de la FHA complète.

Afin de savoir si la FHA tronquée pouvait être produite et sécrétée par d'autres espèces du genre *Bordetella*, pBG4 a été introduit dans *Bordetella parapertussis* PEP (Nordmann, P., François, B., Menozzi, F. D., Commare, M. C., et Barois, A. (1992) *Ped. Infect. Dis. J.* **11,** 248). Le surnageant de culture de la souche transformée a été analysé par SDS-PAGE et le résultat (Fig. 3) indique que *B. parapertussis* peut également sécréter la région N-terminale de la FHA de *B. pertussis*, ce qui suggère que *B. parapertussis* exprime également un gène auxiliaire qui correspond *à fhaC.*

Finalement, pBG4 a été introduit dans la souche *B. pertussis* BPSM et l'analyse du surnageant de culture de la souche *B. pertussis* BPSM (pBG4), appelée *B. pertussis* BPSM4. montre que la production et la sécrétion de la FHA tronquée n'affecte pas la production et la sécrétion de la FHA naturelle et vice versa (Fig. 3). Le système de sécrétion ne semble donc pas être saturé par l'expression du gène *fhaB*.

### IV. Identification du site de fixation à l'héparine et purification de la FHA tronquée sur héparine-sépharose.

La FHA de *B. pertussis* peut interagir avec l'héparine et être purifiée sur héparine-sépharose (Menozzi *et al*., 1991). Afin de savoir si cette interaction implique un site différent de celui qui est responsable de la liaison de la FHA aux intégrines CR3 (Relman et al., 1990) ou de celui responsable de la liaison de la FHA à d'autres glycoconjugués (Prasad *et al*., 1993), différents fragments d'ADN recouvrant ensemble la totalité de la région de *fhaB* qui code pour la FHA mature ont été exprimés en fusion avec MalE (une "maltose-binding protein") en utilisant le système d'expression commercialisé par New England Biolabs (Beverly, MA, USA). Les différents fragments exprimés en fusion avec le gène codant pour MalE sont montrés dans la figure 4. Toutes les protéines de fusion ont été purifiées par chromatographie sur résine d'amylose au départ d'un lysat total obtenu après sonication d'environ 600 ml de culture de *Escherichia coli* TG1 transformé par les différents plasmides recombinants.

Les protéines recombinantes purifiées sur amylose ainsi que MalE et la FHA purifiée ont été chromatographiées sur 3 ml d'héparine-sepharose équilibrés avec 100 ml PBS ("Phosphate Buffered Saline"). 40 ml des différents échantillons ont été préalablement ajustés à une concentration de 5 µg/ml dans PBS + 5 mM maltose, déposés sur la colonne d'héparine sépharose et lavés avec PBS + 5 mM maltose. Les protéines fixées ont alors été éluées avec PBS + 0.5 M NaCl. La quantité des protéines dans la fraction retenue et éluée avec PBS + 0.5 M NaCl et celle des protéines non retenues ont été comparées à celle des protéines totales déposée sur la colonne. L'analyse par SDS-PAGE des différentes fractions a été utilisée pour confirmer que les protéines correspondent bien aux polypeptides de fusion attendus. Les résultats indiquent que seulement le fragment 2 (Fig. 4) code pour un polypeptide qui est significativement retenu sur héparine-sépharose. Puisque le fragment 1 (Fig. 4) ne code pas pour un polypeptide retenu sur héparine-sépharose et puisque ce fragment recouvre partiellement le fragment 2, ceci suggère que la région de la FHA qui interagit avec l'héparine est localisée entre les résidus 441 et 863 si l'on considère la numérotation proposée par Delisse-Gathoye *et al*. (1990). Cette région contient la plupart des "A repeats" et deux "B repeats" (Locht *et al*., 1993) suggérant que l'un ou plusieurs de ces "repeats" pourraient être responsables de la liaison entre la FHA et l'héparine.

La FHA tronquée produite et sécrétée par la souche *B. pertussis* BPGR44 contient la totalité de la région codée par le fragment 2 (Fig. 4) et devrait donc se lier à l'héparine. Le surnageant de culture de cette souche a donc été chromatographié sur héparine-sépharose et élué avec PBS + 0.5 M NaCl. L'analyse par SDS-PAGE et coloration au bleu de Coomassie des différentes fractions protéiques montre que la totalité de la FHA tronquée produite et sécrétée par la souche BPGR44 est retenue sur héparine et peut être purifiée sur héparine-sépharose par une procédure identique à celle utilisée pour la purification de la FHA naturelle (Menozzi *et al*., 1991).

### V. Production et sécrétion de peptides hétérologues en fusion avec la FHA tronquée chez B. pertussis et purification sur héparine-sépharose

Le système efficace de sécrétion de la FHA chez *B. pertussis* a ensuite été utilisé afin de sécréter des peptides hétérologues chez cet organisme. Le peptide modèle utilisé dans cet exemple est celui qui correspond à la région 190-211 de la glutathion-S-transférase de 28 kDa (Sm28GST) de *Schisiosoma mansoni* (Xu, C.-B., Verwaerde, C., Gras-Masse, H., Fontaine, J., Bossus, M., Trottein, F., Wolowczuk, I., Tartar. A., et Capron, A. (1993) *J. Immun.* **150,** 940-949). Deux oligonucléotides synthétiques avec la séquence suivante : 5' TAGGATCCGGGCCGGGGCCCGAAAATCTGTTAGCC 3', et 5' TAAGATCTCCCGGGCCCCGGGAAGGGAGTTGCAGG 3' ont été phosphorylés par des méthodes standard (Sambrook, J., Fritsch, E. F., et Maniatis, T. (1989) Molecular Cloning : A laboratory Manual, 2ième édition, Cold Spring Harbor Laboratories, Cold Spring Harbor, NY), et utilisés pour amplifier par PCR la région codant pour le peptide 190-211. Après amplification, le fragment a été digéré par *Bam*HI et *Bgl*II et inséré dans le site *Bam*HI de pBG4. Les plasmides recombinants ont été analysés par restriction afin de savoir l'orientation de l'insert oligonucléotidique. Un plasmide contenant l'insert oligonucléotidique dans le sens de l'expression de la FHA, appelé pNJ1, a ensuite été purifié et a été introduit dans *B. pertussis* BPGR4 par électroporation. Cette souche recombinante est appelée BPNJ1 et son surnageant de culture a été analysé par SDS-PAGE/coloration au bleu de Coomassie et Western blot en utilisant des anticorps polyclonaux dirigés contre le peptide 190/211 de Sm28GST. Les résultats montrés dans la figure 5 indiquent que la souche BPNJ1 sécrète efficacement le peptide 190/211 de Sm28GST en fusion avec la FHA tronquée et que ce peptide garde son antigénicité.

Le surnageant de culture de la souche BPNJ1 a ensuite été chromatographié sur héparine-sépharose en présence de PBS. L'élution a été réalisée dans PBS + 0.5 M NaCl. L'analyse par SDS-PAGE et coloration au bleu de Coomassie montre que la totalité de la protéine de fusion est retenue sur la colonne et peut être éluée dans PBS + 0.5 M NaCl.

### VI. Stabilité de pNJ1 dans B. pertussis BPNJ1

Afin de connaître la stabilité de pNJ1 dans *B. pertussis* BPNJ1, la souche a été incubée en milieu solide en absence d'antibiotiques. Après 4 jours d'incubation à 37°C, 20 colonies ont été inoculées sur milieu liquide avec et sans kanamycine. Après 4 jours d'incubation à 37°C, le comptage de colonies résistantes à la kanamycine par rapport à celles qui ne sont pas résistantes à la kanamycine indique que toutes les colonies avaient gardé la résistance, montrant que pNJ1 est stable dans *B. pertussis* BPNJ1 en absence de pression sélective. Le contenu en plasmide de 6 colonies résistantes a été analysé par une méthode rapide d'analyse (Baulard, A., Bertin, P., Dartois, V., et Locht, C. (1994) *Meth. Mol. Cell. Biol*. **4,** sous presse) et le résultat montre que toutes les 6 colonies contenaient le plasmide pNJ1.

Cinq souris OF1 ont ensuite été infectées par voie nasale avec *B. pertussis* BPNJ1. Environ 10⁶ cfu ("colony forming units") par souris ont été instillées nasalement. Après 7 jours, les poumons de ces souris ont été prélevés et les *B. pertussis* contenus dans les poumons ont été étalés sur milieu solide avec ou sans kanamycine. Après 4 jours de culture à 37°C, le nombre de bactéries hémolytiques et résistantes à la kanamycine a été comparé au nombre de bactéries hémolytiques et non résistantes à la kanamycine. Le résultat montre qu'environ 95 % des bactéries avaient perdu la résistance à la kanamycine.

Ensemble, ces résultats montrent que pNJ1 est très stable dans *B. pertussis* in vitro mais est relativement instable in vivo.

### VII. Construction de la souche Bordetella pertussis BPGR5, produisant la Sm28GST de S. mansoni en fusion avec la FHA.

Afin de savoir si une protéine hétérologue peut être produite en fusion avec la FHA entière, un fragment contenant l'ADNc complet de la Sm28GST muni de son codon de fin de traduction a été fusionné au cadre de lecture du gène *fhaB* de façon à ce que ce cadre soit interrompu juste derrière l'insertion du gène codant pour la Sm28GST. Un fragment *Bgl*II de 0,68 kb contenant l'ADNc de la Sm28GST a été amplifié par PCR à partir du clone TG10, un dérivé du phage lambda gt10 (Pierce, R., Khalife, J., Williams, D., Kanno, R., Trottein, F., Lepresle, T., Sabatier, J., Achstetter, T., et Capron, A., soumis pour publication) en utilisant les oligonucléotides suivants : 5' TAAGATCTCCATGGCTGGCGAGCAT 3' et 5' TAAGATCTCCGAGCTTTCTGTTG 3'. Après digestion du fragment amplifié par l'enzyme *Bgl*II il a été cloné dans le plasmide pRIT13202 (Delisse-Gathoye *et al*., 1990) préalablement digéré par *Bgl*II et déphosphorylé. Le plasmide recombinant est appelé pUC8-A. Après digestion de pUC8-A par *Eco*RI, le fragment de 10,68 kb est inséré dans le site *Eco*RI du plasmide mobilisable pGR5, un dérivé du plasmide pSS1129 portant les régions flanquantes 5' et 3' du gène *fhaB* (Locht *et al*., 1992). Le plasmide résultant (appelé pGR53) est transféré dans la souche mobilisante E. *coli* S17-1 pour la conjugaison avec *B. pertussis* (Simon, R., Priefer, U., et Puhler, A. (1983) *Bio*/*Technology* **1,** 784-791).

Pour introduire la construction génétique de façon dirigée au locus chromosomique *fhaB*, la souche *E. coli* S17-1(pGR53) est croisée avec *B. pertussis* BPGR4. Cette souche étant dépourvue de la majorité du gène de structure *fhaB*, les événements de double recombinaison homologue sont forcés au niveau des régions flanquantes de ce gène. Après conjugaison, les deux événements de recombinaison sont sélectionnés successivement sur des milieux sélectifs comme décrit précédemment (Antoine, R., et Locht, C. (1990) *Infect. Immun.* **58,** 1518-1526).

Brièvement, *E. coli* S17-1(pGR53) (Sm^{S}, Gen^{R}, Nal^{S}) est croisée sur un milieu solide de Bordet-Gengou (BG) avec *B. pertussis* BPGR4 (Sm^{R}, Gen^{S}, NaI^{R}). Après 6 heures de conjugaison, les transconjuguants sont sélectionnés sur un milieu sélectif (BG + Gentamycine + Acide Nalidixique). La résistance à la gentamycine est amenée par le plasmide pGR53 et celle à l'acide nalidixique est portée par le chromosome de la souche *B. pertussis* BPGR4. Environ 50 colonies isolées sont ensuite purifiées sur le milieu BG + Gentamycine + Acide Nalidixique. Afin de déterminer quel clone Gen^{R} a bien intégré le plasmide pGR53, les transconjuguants sont déposés en réplique sur le milieu sélectif BG + Streptomycine, puis BG + Gentamycine + Acide Nalidixique. Les colonies ayant intégré le plasmide pGR53 sont sensibles à la streptomycine (le caractère de sensibilité à la streptomycine étant dominant sur celui de la résistance). Les transconjuguants Gen^{R}Sm^{S} sont alors étalés sur le milieu sélectif BG + Streptomycine pour sélectionner le second événement de recombinaison correspondant à l'excision du plasmide intégré. Si ce "crossing-over" se réalise au même endroit que la première fois, on retrouve la construction initiale correspondant à la souche BPGR4. Si le second "crossing-over" a lieu dans l'autre région recombinogène, la construction est intégrée dans le génome de *B. pertussis*. L'excision du plasmide ayant apporté la construction est contrôlée par l'apparition du phénotype Sm^{R} Gen^{S}.

Les clones candidats résistants à la streptomycine et portant l'échange allélique voulu sont identifiés par hybridation sur colonies avec une sonde correspondant au fragment *Bgl*II de 0,68 kb décrit plus haut. L'intégrité chromosomique à la jonction des régions flanquantes est confirmée après analyse de l'ADN génomique par Southern blot.

L'antigénicité de la protéine de fusion hétérologue a été mise en évidence par l'analyse en Western blot après séparation par SDS-PAGE en utilisant un gel de 10%. La fraction protéique issue du surnageant de culture et celle associée aux cellules, après croissance des bactéries recombinantes en culture liquide dans du milieu Stainer-Scholtes ont été analysées. La protéine de fusion est identifiée dans les fractions protéiques avec des anticorps polyclonaux dirigés contre la FHA, des anticorps monoclonaux dirigés contre la FHA (Delisse-Gathoye *et al*., 1990), des anticorps polyclonaux de rat dirigés contre la FHA tronquée, des anticorps polyclonaux de lapin dirigés contre la Sm28GST et des anticorps polyclonaux de rat dirigés contre le peptide 190-211 de la Sm28GST. Les anticorps anti-FHA ont été préalablement épuisés contre un lysat total de la souche de *B. pertussis* BPGR4 tandis que les anticorps anti-Sm28GST ont été épuisés contre un lysat total de la souche *B. pertussis* BPSM.

La protéine de fusion est mise en évidence dans la fraction protéique associée aux cellules de la souche BPGR5 où une bande protéique (doublet) d'une taille légèrement supérieure à la FHA réagit à la fois avec des anticorps anti-FHA (fig. 6) et des anticorps anti-Sm28GST (fig. 7). La fusion génétique est donc bien exprimée dans ce nouveau système d'expression hétérologue. Des produits de dégradation (caractéristiques de la FHA) sont également observés dans la fraction protéique associée aux cellules. Cependant, aucun polypeptide n'est immunodétecté dans le surnageant brut de culture, indiquant que la protéine de fusion n'est pas produite efficacement sous une forme sécrétée.

Quand la souche *B. pertussis* BPGR5 est cultivée pendant plus de 48 heures et que le surnageant de cette culture en phase stationnaire est concentré sur une colonne d'héparine-sépharose, un produit de sécrétion est détectable (fig. 8). Il correspond à un produit clivé de la protéine de fusion puisqu'il ne réagit qu'avec les anticorps anti-FHA et pas avec les anticorps anti-Sm28GST. Il paraît donc vraisemblable que dans cette construction la portion de la molécule Sm28GST insérée à l'extrémité de la FHA mature incomplète reste accrochée du côté interne de la membrane externe de la bactérie puisqu'il n'est pas possible de relarguer la protéine de fusion complète dans le surnageant de culture.

### VIII. Construction de la souche B. pertussis BPGR6, produisant une Sm28GST tronquée de S. mansoni en fusion avec la FHA.

Pour mieux exposer l'antigène hétérologue, il nous a semblé plus judicieux de conserver la région de la FHA en aval de l'insertion de la molécule Sm28GST pour ainsi exprimer l'entièreté du précurseur et favoriser l'exportation de la protéine de fusion à travers les deux membranes de *B. pertussis*. Pour cette raison, le cadre de lecture du gène *fhaB* est conservé après l'insertion de l'antigène ou du peptide hétérologue dans les constructions suivantes.

Pour la construction de la souche *B. pertussis* BPGR6, nous avons utilisé un fragment contenant les trois-quarts de l'ADNc de Sm28GST de telle sorte que le cadre de lecture de *fhaB* soit maintenu après l'insertion du gène codant pour une Sm28GST tronquée par délétion de l'extrémité C-terminale du gène. Le gène codant pour la Sm28GST tronquée correspond au fragment *Bgl*II-*Bcl*I de 0,5 kb. Ce fragment a été isolé à partir du fragment *Bgl*II de 0.68 kb et digestion par *Bgl*II et *Bcl*I. Le fragment de 0.5 kb a ensuite été inséré dans le plasmide pRIT13202 (Delisse-Gathoye *et al*., 1990) digéré par *Bgl*II et *Bcl*I éliminant ainsi 0,1 kb du cadre de lecture de *fhaB*. Le plasmide ainsi obtenu est appelé pUC8-F. Le fragment *Eco*RI de 10,4 kb a ensuite été isolé de pUC8-F, inséré dans pGR5 (Locht *et al*., 1992) préalablement digéré par *Eco*RI. Le plasmide pGR54 résultant est ensuite introduit dans la souche *E. coli* S17-1.

La souche E. *coli* S17-1(pGR54) est alors croisée avec *B. pertussis* BPGR4 afin d'intégrer la construction dans le locus *fhaB* chromosomique comme décrit dans l'exemple VII. Après sélection des deux événements de recombinaison et analyse par Southern blot, la souche *B. pertussis* BPGR6 est retenue. Cette souche est donc un dérivé de *B. pertussis* BPSM avec une délétion de 0,1 kb dans le gène *fhaB* et l'insertion chromosomique à cet endroit du gène codant pour la Sm28GST tronquée.

La protéine de fusion est mise en évidence dans la fraction protéique associée aux cellules de la souche BPGR6 où une bande protéique d'une taille légèrement supérieure à la FHA réagit à la fois avec des anticorps anti-FHA (fig. 6) et des anticorps anti-Sm28GST(fig. 7) mais de façon moindre par rapport à la souche BPGR5. Par contre, quand le surnageant d'une culture en phase stationnaire de la souche BPGR6 est concentré sur héparine-sépharose, un produit de sécrétion réagit à la fois avec les anticorps anti-FHA et anti-Sm28GST (fig. 7 et 8), démontrant donc que la protéine de fusion est sécrétée de BPGR6 et/ou présentée à la surface de la bactérie, du côté extérieur. La sécrétion de la protéine de fusion complète reste peu efficace puisque le produit sécrété est majoritairement clivé, comme observé déjà pour la souche BPGR5.

### IX. Construction de la souche Bordetella pertussis BPGR60, produisant une Sm28GST modifiée de S. mansoni en fusion avec la FHA.

La Sm28GST contient une cystéine éventuellement capable de former un pont disulfure. Or, la présence de ponts disulfure peut être un facteur limitant dans l'exportation efficace de protéines dans le surnageant de culture de bactéries gram négatives (Klauser, T., Pohlner, J., et Meyer, T. F. (1990) *EMBO J.* **9**, 1991-1999; Klauser, T., Pohlner, J., et Meyer, T. F. (1992) *EMBO J.* **11**, 2327-2335). Nous avons donc tenté de produire une protéine de fusion entre la FHA et une Sm28GST dont le codon TGC codant pour la cystéine (en position 140 dans la protéine) a été remplacé par le codon AGC codant pour une sérine et où le codon stop de traduction a été éliminé. La construction résultante est donc telle que le cadre de lecture de *fhaB* est maintenu après l'insertion du gène *Sm28* modifié.

Le fragment *Bgl*II-*Sal*I du gène codant pour la Sm28GST modifiée au niveau du codon cystéine a été amplifié par PCR ("polymerase chain reaction"), à l'aide d'amorces spécifiques complémentaires de ces deux régions du gène. Les séquences des oligonucléotides utilisés comme amorces d'amplification sont présentées ci-dessous : sont oligo 5' : 5' TAAGGATCCCCATGGCTGGCGAGCATATCAAG 3' et oligo 3' : 5' CCTGTCGACCCTTTCAGAGATTCGCTGATCATATTGAG 3'. Le produit de 0,44 kb de la PCR a été digéré par *Pst*I et *Sal*I et le fragment de 0,28 kb a été cloné dans le plasmide pUC7-28 préalablement digéré par *Pst*I-*Sal*I, ce qui génère pUC7-28*. Le plasmide pUC7-28 est un dérivé de pUC7 digéré en *Bam*HI (élimination des sites *Pst*I et *Sal*I internes dans pUC7) et ligué avec le fragment *Bam*HI de 0,64 kb provenant de l'amplification par PCR de l'ADNc entier codant pour la Sm28GST. Cette amplification par PCR a été réalisée avec les oligonucléotides suivants : oligo 5' : 5' TAAGGATCCCCATGGCTGGCGAGCATATCAAG 3'; oligo3' : 5' TAAGGATCCCGAAGGGAGTTGCAGGCCTGTT 3'. Les séquences des linkers *Bam*HI ont été choisies de telle manière que ces sites de restriction sont compatibles de chaque côté avec la phase de lecture commençant au site *Bgl*II du gène *fhaB*. Le fragment *Bam*HI du plasmide pUC7-28* est donc isolé et cloné au site *Bgl*II du plasmide pRIT13202, ce qui génère le plasmide pUC8-928*. Ce plasmide est ensuite digéré par *Eco*RI et le fragment *Eco*RI est introduit dans le plasmide pGR5 digéré auparavant par *Eco*RI. Le plasmide pGR540 résultant est ensuite introduit dans la souche d'*E. coli* S17-1.

La souche E. *coli* S17-1(pGR540) est alors croisée avec *B. pertussis* BPGR4 afin d'intégrer la construction dans le locus *fhaB* chromosomique comme décrit dans l'exemple VII. Après sélection des deux événements de recombinaison et analyse par Southern blot, la souche *B. pertussis* BPGR60 est retenue. Cette souche est donc un dérivé de *B. pertussis* BPSM contenant au niveau du site *Bgl*II du gène *fhaB* l'insertion chromosomique du gène codant pour la Sm28GST modifiée.

Dans la souche *B. pertussis* BPGR60, la protéine de fusion est bien visualisée dans la fraction protéique associée aux cellules, où une bande protéique réagit à la fois avec des anticorps anti-FHA (fig. 9) et des anticorps anti-Sm28GST (fig. 10). Dans le surnageant brut, des polypeptides réagissant seulement avec les anticorps anti-FHA sont observés (fig. 9). Quand le surnageant d'une culture en phase stationnaire de cette souche BPGR60 est concentré sur héparine-sépharose, un produit de sécrétion révélé à la fois avec les anticorps anti-FHA et anti-Sm28GST est mis en évidence. Cette reconnaissance est très semblable à celle de la souche BPGR6. La protéine de fusion est donc sécrétée et/ou présentée à la surface de la bactérie, du côté extérieur. Toutefois, la sécrétion de la protéine de fusion complète reste peu efficace et là encore, le produit sécrété est majoritairement clivé.

### X. Etude de la colonisation de la souche recombinante B. pertussis BPGR60 chez la souris après administration par voie nasale.

Afin d'étudier la colonisation de la souche recombinante BPGR60 chez la souris OF1 (souche non pure, souris femelles agées de 4 semaines), 5x10⁶ bactéries en suspension dans du PBS (cellule grattées d'une culture sur milieu solide, Bordet Gengou avec du sang défibriné de mouton (BG); Bordet, J., et Gengou, O. (1906) *Ann. Inst. Pasteur (Paris)* **20,** 731-741) étaient instillées nasalement à raison de 25 µl par narine, sous anesthésie au pentobarbital. Les poumons de 4 à 7 souris étaient prélevés 3 heures après injection, puis à 7, 14, 21, et 28 jours après injection. Les poumons étaient alors homogénéisés dans 5 ml de PBS, puis les bactéries comptées après étalement des broyats sur milieu solide BG contenant 100 µg/ml streptomycine et 25 µg/ml acide nalidixique (BGS₁₀₀N₂₅).

Comme l'indique la figure 11A (ronds pleins), une colonisation au niveau des poumons est observée jusqu'au septième jour, puis est suivie par une chute jusqu'au 28 ième jour où la quasi totalité des bactéries a été éliminée. La cinétique de colonisation de BPGR60 est similaire à celle observée avec la souche sauvage BPSM (carrés vides) à cela près que cette dernière dans cette expérience n'était pas totalement éliminée 28 jours après l'instillation.

Dans le cas où les souris ont reçu préalablement la souche BPGR60 et reçoivent ensuite la souche virulente BPSM, la souche sauvage ne présente plus de phase de croissance, et son élimination se trouve alors accélérée (triangles pleins).

La colonisation de la souche BPGR60 a également été étudiée chez la souris ayant été infectée auparavant par la souche sauvage Tohama I. Ainsi, 28 jours après l'instillation de Tohama I, les souris recevaient une dose de BPGR60, et le nombre des bactéries contenues dans leur poumons a été évalué. Dans ce cas, aucune phase de croissance de la souche BPGR60 n'est observée dans les 7 jours qui suivent l'administration (figure 11B, triangles pleins). Par contre, la chute du nombre de bactéries au cours du temps était plus rapide que celle observée 7 jours après l'administration de BPGR60 chez la souris naïve.

Ces résultats indiquent donc que la souche recombinante se comporte *in vivo* comme la souche sauvage et qu'une primo-infection par l'une des deux souches empêche la colonisation efficace des muqueuses respiratoires par l'autre souche.

### XI. Etude de la production de cytokines inflammatoires consécutive à l'administration nasale de la souche recombinante B. pertussis BPGR60 chez la souris OF1.

La présence de certains micro-organismes ou même de micro-particules peuvent provoquer au niveau des muqueuses pulmonaires, des réactions inflammatoires liées à la présence de cellules capables de produire, après stimulation, des facteurs comme le "tumor necrosis factor" (TNF alpha) ou l'interleukine-6 (Il-6). Ces deux cytokines sont généralement produites localement et sont dosables dans les lavages broncho-alvéolaires. Dans certains cas graves, ces facteurs peuvent être décelés dans le sérum.

De la même façon que précédemment, 5x10⁶ bactéries de la souche recombinante BPGR60 en suspension dans du PBS (cellule grattées d'une culture sur milieu solide BGS₁₀₀N₂₅) étaient instillées nasalement chez la souris OF1 à raison de 25 µl par narine, sous anesthésie au pentobarbital. Des souris recevant des particules de silice ou du PBS étaient utilisées comme témoins. Après 3, 6, 24 heures, puis 3 et 7 jours, la saignée et le lavage broncho-alvéolaire étaient procédés (5 souris par point).

Alors qu'aucune trace de TNF alpha et d'IL-6 n'étaient décelée dans le sang circulant même au delà de 7 jours, des quantités significatives des deux cytokines étaient trouvées dans les lavages broncho-alvéolaires (figure 12).

La production de TNF semblait être immédiate puisque, dès trois heures après injection, le taux maximal de sécrétion était atteint Stable jusqu'à 6 heures au moins après injection, la quantité de TNF alpha était pratiquement nulle 24 heures après et restait négligeable jusqu'au 7ième jour.

La production d'IL-6, très faible les trois premiers jours, augmentait fortement le 3ième jour, puis revenait à un taux normal au septième jour.

Il semble donc que l'administration de BPGR60 induit une réaction inflammatoire localisée, qui est mise en évidence par l'augmentation de TNF et d'IL-6. Cette augmentation de la sécrétion de cytokines inflammatoires était passagère puisque, 7 jours après instillation, les taux normaux de ces cytokines au niveau des poumons étaient retrouvés.

### XII. Réponse immune après administration de la souche recombinante B. pertussis BPGR60 par voie nasale chez la souris OF1.

Les souris femelles OF1 de 4 semaines recevaient 5x10⁶ bactéries en suspension dans du PBS (cellule grattées d'une culture sur milieu solide BGS₁₀₀N₂₅) à raison de 25 µl par narine, sous anesthésie au pentobarbital. La réponse immune était évaluée dans le sang et les liquides broncho-alvéolaires 28, 35, 42, 49 et 56 jours après administration, Au 56 ème jour une partie de ces souris étaient alors restimulée par une nouvelle injection de la souche BPGR60 (mêmes conditions) ou soit par 20 µg d'antigène recombinant purifié (Sm28GST produite chez *E. coli*), par voie nasale. La réponse immune était alors analysée aux jours 70, et 77. Cette réponse immune était comparée soit à celle de souris saines élevées dans les mêmes conditions, soit à celle de souris n'ayant reçu que l'antigène recombinant seul.

Les résultats obtenus dans cet exemple nous indiquent que seule une réponse sérique de type IgA spécifique de l'antigène Sm28GST est observée. Cette réponse anti-Sm28GST, qui ne présente donc aucun anticorps de la classe des IgG, est observée 56 jours après la première injection. Le rappel (J63) n'induit que la persistance de cette réponse sérique IgA spécifique. Par contre, une forte réponse anti-Fha était détectée. Cette réponse, observable dès 28 jours après administration, était maximale après 42 jours et présentait un plateau jusqu'au moment du rappel. Le rappel par la souche BPGR60 (à J63) n'avait pas d'effet sur cette réponse anti-Fha, déjà à son maximum.

Le rappel avec la protéine libre Sm28GST par voie nasale provoque après 8 jours une forte production d'lgG2a et IgG2b. Quinze jours après ce rappel une forte réponse anticorps sérique anti-Sm28GST présentant les isotypes suivants, IgG1, IgG2a, IgG2b et IgA, est obtenue.

Le rappel avec la souche BPGR60 ou la protéine recombinante Sm28GST ne provoquait pas de changements significatifs de la quantité d'IgA sécrétoire anti-Fha, 7 jours après la seconde administration malgré une légère augmentation. Au jour 77 (14 jours après le rappel) le taux d'anti-Fha diminuait dans les deux groupes.

Par contre le rappel par BPGR60 induisait, 7 jours après, une augmentation de la quantité d'anticorps sécrétoires spécifiques de la Sm28GST qui diminuait 14 jours après. Le rappel avec la protéine recombinante provoquait une forte augmentation du taux d'IgA sécrétoires spécifiques, plus importante que celle obtenue avec la souche BPGR60, mais assez hétérogène. Cette augmentation d'IgA anti Sm28GST n'était pas durable puisque dès 14 jours, ce taux chutait et retrouvait les valeurs obtenues juste avant le rappel.

La recherche de complexes antigène-anticorps de type IgA-Sm28GST dans les lavages broncho-alvéolaires avant et après rappel avec la souche BPGR60, a été effectuée. Les résultats obtenus, et présentés dans la figure 14, nous montrent qu'une grande proportion des IgA anti-Sm28GST se présentent sous forme de complexes. Cet exemple nous indique que l'utilisation de BPGR60 comme formulation de rappel est parfaitement adaptée pour l'obtention d'un effet de restimulation de la réponse immune vis-à-vis de l'antigène étranger.

Aucune production d'anticorps IgA anti-Sm28GST ou anti-Fha n'était détectée dans les sécrétions broncho-alvéolaires de souris n'ayant reçu que la Sm28GST au moment du rappel.

L'administration par voie nasale de la souche BPGR60 exprimant la Sm28GST est donc capable d'induire une réponse immune sécrétoire vis-à-vis de cet antigène. Cette réponse anticorps peut être amplifiée par le rappel soit avec la souche recombinante, soit avec la Sm28GST seule. Ce mode de vaccination pourrait être sans doute amélioré en différant le temps écoulé entre l'immunisation et le rappel (exemple : 90 jours au lieu de 56). Les quantités de bactéries et la dose de protéines peuvent être considérées comme optimales.

### XIII. Etude de l'effet protecteur de l'immunisation par BPGR60 sur la charge parasitaire de souris infectées par S.mansoni

Précédemment à l'infection, des souris femelles OF1 ont été immunisées conformément au protocole indiquée dans l'exemple précédent et subirent un rappel par la protéine libre (J63). Quinze jours suivant le rappel, ces souris furent infestées par 80 cercaires (voie transcutanée, abdomen). La charge parasitaire a été ensuite évaluée 42 jours après l'infestation, par la charge vermineuse et par la charge en oeufs hépatiques et intestinaux. Les résultats obtenus sont comparés à ceux obtenus dans la même expérience chez des souris non traitées ou n'ayant reçues qu'une injection de Sm28GST au moment du rappel.

Alors que l'injection unique de Sm28GST ne provoque pas d'effets significatifs sur la charge parasitaire, l'immunisation à l'aide de la souche BPGR60 induit une protection significative contre l'infection à S. mansoni que cela soit au niveau de la charge vermineuse (Fig. 15A) que de la charge en oeufs (Fig. 15B). Ce résultat indique qu'une souche recombinante de B.pertussis exprimant une protéine étrangère en fusion avec la Fha présente des qualités vaccinales.

### XIV. Production et sécrétion de la FHA tronquée chez Escherichia coli.

En vue de la sécrétion de la FHA tronquée dans *E. coli*, les gènes codant la protéine accessoire FhaC et la protéine FHA44 (codée initialement par pBG4) ont été placés sous le contrôle du promoteur *tac* afin de tirer parti d'une expression régulable et de se débarrasser du système de régulation de l'expression de FHA chez *B.pertussis*. Le fragment *Bcl*1 de 2.3 kbp de pRIT12990 (Delisse-Gathoye et al., 1990) contenant l'extrémité 3' du gène *fimD* et la totalité du gène *fhaC* a été cloné dans pQE32 (Qiagen, Hilden, Allemagne) préalablement digéré par *Bam*H1 de façon à générer une fusion traductionnelle entre la séquence du vecteur codant un motif polyhistidine et l'extrémité 3' de *fimD*. Grâce à cette disposition, la transcription de ce gène hybride et de *fhaC* se trouve sous le contrôle du promoteur *tac* inductible à l'IPTG. Par ailleurs, la traduction des 2 gènes, dont l'extrémité 3' du premier chevauche l'extrémité 5' du second, peut se dérouler de façon couplée. Ce plasmide est appelé pFJD6. Une culture liquide de *E. coli* XL1 blue transformée par ce plasmide est traitée avec 1 mM d'IPTG pendant deux heures après avoir atteint une absorbance de 0.8 à 600 nm. Les cellules sont ensuite collectées, lysées par sonication et les membranes cellulaires sont obtenues par ultracentrifugation (15°C, 60 min 100.000 x g) du sonicat clarifié. Une extraction au sarkosyl (1%) solubilise sélectivement la membrane cytoplasmique et une deuxième ultracentrifugation (15°C, 60 min 100.000 x g) permet d'obtenir une fraction enrichie en protéines de la membrane externe. Leur analyse par électrophorèse en gel de polyacrylamide en présence de SDS et immunoblotting avec un sérum polyclonal dirigé contre FhaC montre qu'une protéine de la même taille que FhaC de *B. pertussis* est présente dans ces extraits, tandis que la souche non transformée ne la possède pas. Ces observations indiquent que *E. coli* est capable de produire et localiser correctement FhaC dans la cellule.

Le fragment *EcoR*1-*Bam*H1 codant la FHA tronquée (appelée FHA44) de pBG4 a été cloné dans les mêmes sites du vecteur pMMB91 (P.J. Fürste, W; Pansegrau, R. Frank, H. Blöcker, P. Scholz, M. Bagdasarian and E. Lanka, Gene 48, 119-131, 1986) sous le contrôle du promoteur *tac*. Le plasmide résultant, pFJD9, a été introduit par transformation dans les souches d'*E*. *coli* XL1 blue et UT5600 (cette dernière ne produit pas la protéase d'enveloppe OmpT), seul ou *en trans* avec pFJD6. Les deux plasmides possèdent des origines de réplication compatibles et des marqueurs de résistance différents. Un immunoblot réalisé sur des colonies possédant les deux plasmides repiquées sur milieu solide contenant 50 µM d'IPTG montre que 1a surface des cellules est reconnue par un antisérum dirigé contre 1a FHA tronquée alors qu'aucune réactivité n'est détectée à la surface des cellules possédant un seul des deux plasmides. Par contre, le fractionnement des cellules et l'analyse des protéines par immunoblotting avec le même antiserum ne permet pas de détecter de bande protéique correspondant à FHA44, ce qui suggère que la protéine est faiblement produite ou fortement dégradée. En culture liquide on n'obtient pas non plus de quantités détectables de FHA tronquée dans le surnageant de culture ou associé aux cellules.

La région extrême aminoterminale de FHA ne possède guère les caractéristiques d'un peptide signal classique et pourrait entraver la sécrétion efficace de FHA chez *E. coli*. Différentes constructions ont été réalisées afin d'y ajouter la préséquence codant le peptide signal de la préprotéine preOmpA d'*E. coli* . Cette préséquence a été greffée en trois endroits différents de la région 5' du gène *fha* tronqué. La région 5' du gène a été amplifiée par PCR à partir du plasmide pRIT13130 (Delisse-Gathoye et al.,1990) en utilisant l'oligonucléotide (5'-3') TTTAACCGATGCGGCCGCCGTTG qui contient un site *Not*1 (souligné) et chacun des trois oligonucléotides (5'-3') TATAAGCTTCGAACCTGTACAGGCTGGTC,TCAAAGCTTCGCGTGGTCAAGCG CGAAG, et ATTAAGCTTCCCAGGGCTTGGTTCCTCAG, renfermant des sites Hind3 (soulignés). Le fragment de 100 bp *Xba*1*-Bam*H1 de pIN-OmpAIII-Hind (F. Rentier-Delrue, D. Swennen et J. Martial, Nucleic Acid Res. 16, 8726, 1988) contenant la préséquence de preOmpA a été cloné dans pACYC184, donnant le plasmide pEC1. Les trois produits PCR de 615 bp, 520 bp et 410 bp ont ensuite été purifiés et digérés par *Not*1 et *Hind3* et chacun d'entre eux a été utilisé dans une ligation à trois partenaires avec pEC1 restreint par *Hind3* et *Bam*H1 et le fragment *Not*1-*Bam*H1 de 2 kb de pRIT13197 codant la région 5' de FHA44 (Delisse-Gathoye et al., 1990). Les trois plamides résultants, PEC11, pEC12 et pEC13 ont été digérés par *Xba*1, le site a été rempli à la Klenow et les plasmides ont été redigérés par *Bam*H1. Les fragments d'ADN contenant les gènes chimères préOmpA-fha44 ont été clonés dans les sites *Eco*R1 (rempli à la Klenow) et *Bam*H1 de pMMB91. Les plasmides résultants, pFJD11, pFJD12 et pFJD13, codent des gènes chimères dont les produits sont respectivement des protéines de fusion dans lesquelles les 2, 33 ou 71 premiers acides aminés de FHA44 sont remplacés par les 18 acides aminés du peptide signal de preOmpA. Les plasmides ont été introduits dans la souche UT5600 seuls ou en trans avec pFJD6 et l'expression de *fhaC* et des gènes chimères a été induite par l'ajout d'IPTG à des cultures liquides ayant atteint une absorbance de 1 à 600 nm. Les cultures ont été incubées pour trois heures en présence d'IPTG (1 mM final). FHA44 est détecté par immunobloting après électrophorèse en gel de polyacrylamide en présence de SDS dans les surnageants de cultures provenant des souches exprimant FhaC. La production de FHA44 est assez faible dans les souches UT5600(pFJD6, pFJD11) et UT5600(pFJD13, pFJD6) mais nettement plus élevée dans la souche UT5600(pFJD12, PFJD6), qui contient le gène chimérique de longueur intermédiaire. La production de FHA44 dans le surnageant de cette souche est estimée par ELISA et immunoblotting à environ 20 % de la production de FHA tronquée chez *B. pertussis* (BPGR44). En revanche les deux autres souches sécrètent 8 à 10 fois moins de FHA44 que la souche UT5600(pFJD12, pFJD6). En absence de FhaC, la sécrétion est négligeable pour UT5600(pFJD11) et UT5600(pFJD13) et très faible pour UT5600(pFJD12), ce qui indique que la FHA tronquée est sécrétée chez *E. coli* par sa protéine accessoire et n'est pas relarguée de façon non spécifique. FHA44 sécrétée par *E. coli* a la même taille que celle de *B. pertussis* et elle est purifiable dans les mêmes conditions par chromatographie d'affinité sur héparine-sépharose. Le séquençage aminoterminal des deux protéines montre qu'elles subissent la même maturation aminoterminale.

Cet exemple montre qu'il est possible de faire sécréter eficacement la FHA tronquée indistingable de celle produite par *B. pertussis* chez d'autres microorganismes Gram négatifs, pathogènes ou non pathogènes, moyennant le transfert de l'apareillage de sécrétion - FhaC semble suffisant- et le remodelement de la région amino-terminale de la FHA. Il est donc probable que des constructions similaires pourront être utilisées pour exprimer et sécréter la FHA entière ou tronquée pourvu que les séquences de sécrétions soient fonctionnellement présentes. Cette technologie est susceptible d'être également étendue à d'autres bactéries Gram négatives, telles que Salmonella, Vibrio, et d'autres.

## Revendications

1. ADN recombinant contenant une séquence (1) codant pour un polypeptide hétérologue vis-à-vis d'une hémagglutinine filamenteuse de Bordetella (Fha) fusionnée dans le même cadre de lecture à une séquence (2) placée en amont de la première, cette séquence (2) codant pour une partie au moins du précurseur de la Fha, cette partie comportant au moins la région N-terminale d'une protéine mature tronquée de Fha qui contient le site d'interaction de la Fha avec l'héparine, d'une part, et qui, lorsque celle-ci est elle-même placée, seule, sous le contrôle d'un promoteur reconnu par les polymérases cellulaires de B.pertussis et introduite dans une culture de cellules de B.pertussis, est exprimée dans cette culture sous le contrôle de ce promoteur et excrétée dans le milieu de culture de ces cellules, d'autre part.

2. ADN recombinant selon la revendication 1, **caractérisé en ce que** la Fha est une Fha de B.pertussis.

3. ADN recombinant selon la revendication 1 ou 2, **caractérisé en ce que** la séquence (2) code pour la protéine mature de Fha.

4. ADN recombinant selon la revendication 1 ou 2, **caractérisé en que** la séquence (2) résulte d'une troncature de la séquence codant pour la protéine mature Fha du côté de son extrémité C-terminale.

5. ADN recombinant selon l'une quelconque des revendications 1 à 4, **caractérisé en ce qu'**il comporte en outre une séquence (3) en aval de la séquence (1), cette séquence (3) correspondant pour l'essentiel à la partie tronquée de la protéine mature, de préférence complétée par la séquence signal du précurseur.

6. ADN recombinant selon l'une quelconque des revendications 1 à 4, **caractérisé en ce que** la séquence (2) comporte les signaux d'excrétion de la séquence codant pour la Fha et la région N-terminale homologue aux régions N-terminales des hémolysines ShIA et HpmA de Serratia marcescens et Proteus mirabilis.

7. ADN recombinant selon la revendication 4 ou 6, **caractérisé en ce que** l'extension de la séquence (2) du côté de son extrémité C-terminale ne va pas au-delà de la longueur qui ferait que la transformation de B.pertussis avec cet ADN recombinant alors placé sous le contrôle d'un promoteur susceptible d'être reconnu par B.pertussis ne permette plus l'excrétion directe de la protéine recombinante alors formée dans le milieu de culture de ce B.pertussis.

8. ADN recombinant selon la revendication 6 ou 7, **caractérisé en ce que** la séquence (2) s'étend entre l'ATG correspondant au codon d'initiation de la traduction de la Fha à un nucléotide C-terminal au delà du nucléotide 907 dans la direction de la traduction et de préférence non au delà de la position 6922.

9. ADN recombinant selon la revendication 8, **caractérisé en ce qu'**il ne réagit plus avec des anticorps anti-Fha plus particulièrement dirigés contre les épitopes de la partie C-terminale de la Fha mature, situés au delà du site nucléotidique 2841 dans le sens de la traduction.

10. ADN recombinant selon l'une quelconque des revendications 1 à 8, **caractérisé en ce que** le polypeptide codé par la séquence (2) contient au moins un site spécifique d'attachement de la Fha aux muqueuses.

11. ADN recombinant selon l'une quelconque des revendications 1 à 10, **caractérisé en ce que** la séquence (1) code pour un polypeptide ayant des propriétés vaccinantes vis-à-vis d'un agent pathogène donné.

12. ADN recombinant selon l'une quelconque des revendications 1 à 11, **caractérisé en ce qu'**il contient également un promoteur reconnu par les polymérases d'une cellule transformable avec un vecteur contenant l'ADN recombinant en question et autorisant l'expression des séquences (1) et (2), dès lors que se trouve également exprimé dans cette cellule un gène auxiliaire du type fhaC.

13. ADN recombinant selon la revendication 12, **caractérisé en ce que** le promoteur est un promoteur reconnu par les polymérases d'une bactérie de l'espèce Bordetella, notamment de B.pertussis qui dans le produit naturel régule l'expression de la protéine Fha.

14. Culture de cellules procaryotes, notamment bactéries, transformées avec un ADN recombinant selon la revendication 11 ou 12, **caractérisée en ce que** le promoteur de l'ADN recombinant est reconnu par les polymérases de ladite cellule procaryote.

15. Culture selon la revendication 14, **caractérisée en ce que** les cellules appartiennent à une espèce Bordetella, notamment B.pertussis, et qu'elles sont également porteuses d'un gène fhaC exprimable dans ces cellules.

16. Culture selon la revendication 14, **caractérisée en ce que** les cellules appartiennent à une espèce bactérienne autre que Bordetella et qu'elles contiennent également une séquence codant pour au moins la partie de FhaC nécessaire à l'expression de la séquence (2), sous une forme également exprimable au sein des cellules de cette culture.

17. Culture cellulaire selon la revendication 16, **caractérisée en ce que** ses cellules appartiennent à l'espèce E.coli.

18. Culture cellulaire selon l'une quelconque des revendications 14 à 17, **caractérisée en ce que** l'ADN recombinant est incorporé à l'ADN chromosomique desdites cellules.

19. Culture de cellules selon l'une quelconque des revendications 14 à 18, **caractérisée par** l'exposition du produit d'expression de la séquence (1) à leur surface.

20. Culture selon l'une quelconque des revendications 14 à 19,
**caractérisée en ce que** la séquence (2) contient au moins un site d'adhésion de la Fha aux muqueuses ou à des cellules eucaryotes, notamment macrophages ou cellules épithéliales.

21. Culture cellulaire selon la revendication 20, **caractérisée en ce qu'**elle est détoxifiée ou atténuée.

22. Composition immunogène orientée contre un agent pathogène déterminé et **caractérisée en ce qu'**il contient à titre de principe actif, des cellules de la culture selon l'une quelconque des revendications 18 à 21 dans lesquelles la séquence (1) code pour un antigène caractéristique de cet agent pathogène.

23. Protéine recombinante constituée par le produit d'expression de l'ADN recombinant selon l'une quelconque des revendications 1 à 13.

24. Protéine recombinante selon la revendication 23, **caractérisée par le fait qu'**elle comporte l'un au moins des sites d'adhésion de la protéine Fha aux muqueuses.

25. Protéine recombinante selon la revendication 24, **caractérisée en ce que** le produit d'expression de la séquence (1) code pour un polypeptide ayant des propriétés vaccinantes vis-à-vis d'un agent pathogène donné et **en ce que** le produit d'expression de la séquence (2) contient un site d'adhésion de la Fha aux muqueuses ou à des cellules eucaryotes, notamment macrophages ou cellules épithéliales.

26. Composition vaccinante contenant la culture cellulaire de la revendication 22 ou la protéine recombinante de la revendication 25, **caractérisée en ce qu'**elle présente l'immunogénicité mucosale de la Fha, notamment pour l'administration par voie nasale.

27. Procédé pour la production d'une protéine hétérologue recombinante contenant une séquence polypeptidique déterminée **caractérisée par** la transformation d'une culture de cellules procaryotes avec un vecteur contenant un ADN recombinant conforme à l'une quelconque des revendications 1 et 6 à 13, lesdites cellules procaryotes contenant également une séquence nucléotidique codant pour FhaC sous une forme susceptible d'y être exprimée ou ayant également été transformées à cette fin, puis la culture de ces cellules et la récupération du produit excrété par les cellules de cette culture dans leur milieu.

28. Procédé selon la revendication 27, **caractérisé en ce que** lesdites cellules procaryotes sont des Bordetella, notamment du type B.pertussis.

29. Procédé selon la revendication 27 ou 28, **caractérisé par** la purification supplémentaire du produit d'excrétion par mise en contact du milieu de culture avec de l'héparine immobilisée sur un support insoluble et par récupération de la protéine recombinante purifiée par dissociation du complexe qu'il formait avec l'héparine.

## Patentansprüche

1. Rekombinante DNA umfassend eine Sequenz (1), die ein bezüglich eines filamentösen Hämagglutinins von Bordetella (Fha) heterologes Polypeptid codiert, fusioniert im gleichen Leseraster an eine Sequenz (2), welche stromaufwärts von der ersten Sequenz liegt, wobei diese Sequenz (2) zumindest einen Teil des Vorläufers des Fha codiert, wobei dieser Teil mindestens den N-terminalen Bereich eines verkürzten reifen Proteins von Fha umfasst, der die Interaktionsstelle des Fha mit Heparin enthält, und welche, wenn sie alleine unter die Kontrolle eines Promotors gestellt ist, der durch die zellulären Polymerasen von B. pertussis erkannt wird, und in eine Zellkultur von B. pertussis eingeführt ist, in dieser Kultur unter der Kontrolle dieses Promotors exprimiert wird und in das Kulturmedium dieser Zellen ausgeschieden wird.

2. Rekombinante DNA nach Anspruch 1, **dadurch gekennzeichnet, dass** Fha ein Fha von B. pertussis ist.

3. Rekombinante DNA nach Anspruch 1 oder 2, **dadurch gekennzeichnet, dass** die Sequenz (2) das reife Protein von Fha codiert.

4. Rekombinante DNA nach Anspruch 1 oder 2, **dadurch gekennzeichnet, dass** die Sequenz (2) aus einer Verkürzung der Sequenz resultiert, die das reife Protein Fha am C-terminalen Ende codiert.

5. Rekombinante DNA nach einem der Ansprüche 1 bis 4, **dadurch gekennzeichnet, dass** sie weiterhin eine Sequenz (3) stromabwärts der Sequenz (1) umfasst, wobei die Sequenz (3) im wesentlichen einem verkürzten Teil des reifen Proteins entspricht, vorzugsweise ergänzt durch die Signalsequenz des Vorläufers.

6. Rekombinante DNA nach einem der Ansprüche 1 bis 4, **dadurch gekennzeichnet, dass** die Sequenz (2) die Ausscheidungssignale der Sequenz umfasst, die das Fha und den N-terminalen Bereich codiert, welcher homolog zu den N-terminalen Bereichen des Hämolysins ShlA und HpmA von Serratia marcescens und Proteus mirabilis ist.

7. Rekombinante DNA nach Anspruch 4 oder 6, **dadurch gekennzeichnet, dass** die Verlängerung der Sequenz (2) an ihrem C-terminalen Ende nicht über die Länge hinausgeht, die dazu führen würde, dass die Transformation von B. pertussis mit dieser rekombinanten DNA, welche unter die Kontrolle eines Promotors gestellt ist, welcher von B. pertussis erkannt werden kann, nicht mehr die direkte Ausscheidung des rekombinanten Proteins erlaubt, das sich in dem Kulturmedium dieses B. pertussis dann gebildet hat.

8. Rekombinante DNA nach Anspruch 6 oder 7, **dadurch gekennzeichnet, dass** die Sequenz (2) sich zwischen dem ATG, welches dem Initiationscodon der Translation des Fha entspricht, und einem C-terminalen Nucleotid außerhalb des Nucleotids 907 in Translationsrichtung und vorzugsweise nicht außerhalb der Position 6922 befindet.

9. Rekombinante DNA nach Anspruch 8, **dadurch gekennzeichnet, dass** sie nicht mehr mit anti-Fha-Antikörpern reagiert, welche insbesondere gegen die Epitope des C-terminalen Teils des reifen Fha gerichtet sind, welche sich außerhalb der Nucleotidstelle 2841 in Translationsrichtung befinden.

10. Rekombinante DNA nach einem der Ansprüche 1 bis 8, **dadurch gekennzeichnet, dass** das von Sequenz (2) codierte Polypeptid mindestens eine spezifische Anheftungsstelle des Fha an die Schleimhäute enthält.

11. Rekombinante DNA nach einem der Ansprüche 1 bis 10, **dadurch gekennzeichnet, dass** die Sequenz (1) ein Polypeptid codiert, welches Impfeigenschaften bezüglich eines gegebenen pathogenen Agens besitzt.

12. Rekombinante DNA nach einem der Ansprüche 1 bis 11, **dadurch gekennzeichnet, dass** sie ebenfalls einen Promotor enthält, welcher erkannt wird durch die Polymerasen einer Zelle, die mit einem Vektor transformierbar ist, welcher die fragliche rekombinante DNA enthält und die Expression der Sequenzen (1) und (2) ermöglicht, sobald ebenfalls in dieser Zelle ein zusätzliches Gen des Typs fhaC exprimiert wird.

13. Rekombinante DNA nach Anspruch 12, **dadurch gekennzeichnet, dass** der Promotor ein Promotor ist, welcher durch die Polymerasen eines Bakteriums der Art Bordetella erkannt wird, insbesondere von B. pertussis, welche in dem natürlichen Produkt die Expression des Proteins Fha reguliert.

14. Kultur von prokaryontischen Zellen, insbesondere Bakterien, transformiert mit einer rekombinanten DNA nach Anspruch 11 oder 12, **dadurch gekennzeichnet, dass** der Promotor der rekombinanten DNA von den Polymerasen der prokaryontischen Zelle erkannt wird.

15. Kultur nach Anspruch 14, **dadurch gekennzeichnet, dass** die Zellen einer Bordetella-Art angehören, insbesondere B. pertussis und dass sie ebenfalls Träger eines Gens fhaC sind, welches in diesen Zellen exprimiert werden kann.

16. Kultur nach Anspruch 14, **dadurch gekennzeichnet, dass** die Zellen einer anderen Bakterienart als Bordetella angehören und dass sie ebenfalls eine Sequenz erhalten, die mindestens einen Teil des FhaC codieren, welcher für die Expression der Sequenz (2) wichtig ist, dergestalt, dass die Sequenz ebenfalls in den Zellen dieser Kultur exprimiert ist.

17. Zellkultur nach Anspruch 16, **dadurch gekennzeichnet, dass** ihre Zellen der Art E. coli angehören.

18. Zellkultur nach einem der Ansprüche 14 bis 17, **dadurch gekennzeichnet, dass** die rekombinante DNA in die Chromosomen-DNA der Zellen eingebaut ist.

19. Zellkultur nach einem der Ansprüche 14 bis 18, **gekennzeichnet durch** die Exposition des Expressionsprodukts der Sequenz (1) an der Zelloberlläche.

20. Kultur nach einem der Ansprüche 14 bis 19, **dadurch gekennzeichnet, dass** die Sequenz (2) mindestens eine Adhäsionsstelle des Fha an die Schleimhäute enthält oder an eukaryontische Zellen, insbesondere Makrophagen oder epitheliale Zellen.

21. Zellkultur nach Anspruch 20, **dadurch gekennzeichnet, dass** sie entgiftet oder attenuiert ist.

22. Immunogene Zusammensetzung, der gegen ein bestimmtes pathogenes Agens gerichtet ist und **dadurch gekennzeichnet ist, dass** er als aktiven Wirkstoff Zellen der Kultur nach einem der Ansprüche 18 bis 21 enthält, wobei die Sequenz (1) ein für dieses pathogene Agens charakteristisches Antigen codiert.

23. Rekombinantes Protein, bestehend aus dem Expressionsprodukt der rekombinanten DNA nach einem der Ansprüche 1 bis 13.

24. Rekombinantes Protein nach Anspruch 23, **dadurch gekennzeichnet, dass** es mindestens eine der Adhäsionsstellen des Fha-Proteins an die Schleimhäute umfasst.

25. Rekombinantes Protein nach Anspruch 24, **dadurch gekennzeichnet, dass** das Expressionsprodukt der Sequenz (1) ein Polypeptid codiert, welches Impfeigenschaften gegenüber einem gegebenen pathogenen Agens hat und **dadurch gekennzeichnet, dass** das Expressionsprodukt der Sequenz (2) eine Adhäsionsstelle des Fha an die schleimhäute enthält oder an eukaryontische Zellen, insbesondere Makrophagen oder epitheliale Zellen.

26. Impfstoff enthaltend die Zellkultur des Anspruchs 22 oder das rekombinante Protein des Anspruchs 25, **dadurch gekennzeichnet, dass** er die Schleimhautimmunogenität des Fha präsentiert, insbesondere bei nasaler Verabreichung.

27. Verfahren zur Herstellung eines heterologen rekombinanten Proteins enthaltend eine bestimmte Polypeptidsequenz, **gekennzeichnet durch** die Transformation einer prokaryontischen Zellkultur mit einem Vektor, der eine rekombinante DNA nach einem der Ansprüche 1 und 6 bis 13 enthält, wobei die prokaryontischen Zellen ebenfalls eine Nucleotidsequenz enthalten, die FhaC codiert, die in einer Form vorliegt, so dass die Sequenz exprimiert werden kann oder wobei die Zellen zu diesem Zweck transformiert worden; anschließend die Züchtung dieser Zellen und die Gewinnung des von den Zellen dieser Kultur in ihr Medium ausgeschiedenen Produktes.

28. Verfahren nach Anspruch 27, **dadurch gekennzeichnet, dass** die prokaryontischen Zellen von Bordetella stammen, insbesondere vom Typ B. pertussis.

29. Verfahren nach Anspruch 27 oder 28, **gekennzeichnet durch** die zusätzliche Reinigung des Ausscheidungsproduktes **durch** Inkontaktbringen des Kulturmediums mit dem auf einem unlöslichen Träger immobilisierten Heparin und Gewinnung des rekombinanten Proteins, das **durch** Dissoziation des Komplexes, welchen er mit dem Heparin formte, gereinigt wird.

## Claims

1. Recombinant DNA containing a sequence (1) coding for a polypeptide that is heterologous as regards a filamentous haemagglutinin of Bordetella (Fha) fused in the same reading frame to a sequence (2) located upstream of the first sequence, said sequence (2) coding for at least a portion of a Fha precursor, said portion comprising at least the N-terminal region of a mature truncated Fha protein that contains the Fhaheparin interaction site and that, when it is placed alone under the control of a promoter recognized by cellular polymerases of B. pertussis and introduced into a B. pertussis cell culture, is expressed in said culture under the control of said promoter and excreted into the culture medium for said cells.

2. Recombinant DNA according to claim 1, **characterized in that** the Fha is an Fha from B. pertussis.

3. Recombinant DNA according to claim 1 or claim 2, **characterized in that** sequence (2) codes for the mature Fha protein.

4. Recombinant DNA according to claim 1 or claim 2, **characterized in that** sequence (2) results from truncating the sequence coding for the mature Fha protein on its C-terminal side.

5. Recombinant DNA according to any one of claims 1 to 4, **characterized in that** it further comprises a sequence (3) downstream of sequence (1), said sequence (3) essentially corresponding to the truncated portion of the mature protein, preferably completed by the signal sequence for the precursor.

6. Recombinant DNA according to any one of claims 1 to 4, **characterized in that** sequence (2) comprises the excretion signals for the sequence coding for Fha and the N-terminal region that is homologous with the N-terminal regions of the haemolysins ShIA and HpmA from Serratia marcescens and Proteus mirabilis.

7. Recombinant DNA according to claim 4 or claim 6, **characterized in that** the extension of sequence (2) on its C-terminal side does not extend beyond the length that would cause transformation of B. pertussis with this recombinant DNA when placed under the control of a promoter that is capable of being recognized by B. pertussis to no longer allow direct excretion of the recombinant protein formed in the B. pertussis culture medium.

8. Recombinant DNA according to claim 6 or claim 7, **characterized in that** sequence (2) extends between the ATG corresponding to the start codon for Fha translation to a C-terminal nucleotide beyond nucleotide 907 in the direction of translation and preferably not beyond position 6922.

9. Recombinant DNA according to claim 8, **characterized in that** it no longer reacts with anti-Fha antibodies, more particularly those directed against epitopes for the C-terminal portion of mature Fha, located beyond nucleotide site 2841 in the direction of translation.

10. Recombinant DNA according to any one of claims 1 to 8, **characterized in that** the polypeptide coded by sequence (2) contains at least one specific site for attachment of Fha to the mucosa.

11. Recombinant DNA according to any one of claims 1 to 10, **characterized in that** sequence (1) codes for a polypeptide having vaccine properties as regards a given pathogenic agent.

12. Recombinant DNA according to any one of claims 1 to 11, **characterized in that** it also contains a promoter recognized by the polymerases of a cell that is transformable with a vector containing the recombinant DNA in question and authorizing the expression of sequences (1) and (2), provided that an auxiliary fhaC type gene is also expressed in said culture.

13. Recombinant DNA according to claim 12, **characterized in that** the promoter is a promoter recognized by the polymerases of a bacteria from the Bordetella species, in particular B. pertussis, which regulates the expression of the Fha protein in the natural product.

14. A prokaryotic cell culture, in particular of bacteria, transformed with a recombinant DNA according to claim 11 or claim 12, **characterized in that** the promoter for the recombinant DNA is recognized by the polymerases of said prokaryotic cell.

15. A culture according to claim 14, **characterized in that** the cells belong to a Bordetella species, in particular B. pertussis, and **in that** they also carry a fhaC gene that can be expressed in said cells.

16. A culture according to claim 14, **characterized in that** the cells belong to a bacterial species other than Bordetella and **in that** they also contain a sequence coding for at least the portion of the FhaC necessary for expression of sequence (2), in a form that is also expressible in the cells of said culture.

17. A cell culture according to claim 16, **characterized in that** its cells belong to the species E. coli.

18. A cell culture according to any one of claims 14 to 17, **characterized in that** the recombinant DNA is incorporated into the chromosomic DNA of said cells.

19. A cell culture according to any one of claims 14 to 18, **characterized by** exposure of the expression product of sequence (1) on their surface.

20. A culture according to any one of claims 14 to 19, **characterized in that** the sequence (2) contains at least one site for adhesion of Fha to the mucosa or to eukaryotic cells, in particular macrophages or epithelial cells.

21. A cell culture according to claim 20, **characterized in that** it is detoxified or attenuated.

22. An immunogenic composition orientated against a given pathogenic agent and **characterized in that** it contains, as the active principle, cells of a culture according to any one of claims 18 to 21 in which sequence (1) codes for an antigen that is characteristic of said pathogenic agent.

23. A recombinant protein constituted by the expression product of a recombinant DNA according to any one of claims 1 to 13.

24. A recombinant protein according to claim 23, **characterized in that** it comprises at least one site for adhesion of the Fha protein to the mucosa.

25. A recombinant protein according to claim 24, **characterized in that** the expression product of sequence (1) codes for a polypeptide having vaccine properties as regards a given pathogenic agent and **in that** the expression product of sequence (2) contains a site for adhesion of Fha to mucosa or to eukaryotic cells, in particular to macrophages or epithelial cells.

26. A vaccine composition containing the cell culture of claim 22 or the recombinant protein of claim 25, **characterized in that** it has mucosal Fha immunogenicity, in particular for nasal administration.

27. A process for producing a recombinant heterologous protein containing a set polypeptide sequence, **characterized by** transforming a prokaryotic cell culture with a vector containing a recombinant DNA according to any one of claims 1 and 6 to 13, said prokaryotic cells also containing a nucleotide sequence coding for FhaC in a form that can be expressed therein or also having been transformed to this end, then culturing said cells and recovering the product excreted by the cells of said culture into their medium.

28. A process according to claim 27, **characterized in that** said prokaryotic cells are Bordetella, in particular of the B. pertussis type.

29. A process according to claim 27 or claim 28, **characterized by** supplemental purification of the excretion product by bringing the culture medium into contact with heparin immobilized on an insoluble support and by recovery of the purified recombinant product by dissociation of the complex that it forms with the heparin.
